# EUROPEAN PATENT APPLICATION

(11) **EP 4 803 507 A1**
(43) Date of publication of application: **09.09.2026**
(21) Application number: 24885601.5
(22) Date of filing: 24.10.2024
(51) Int. Cl.: C07C 381/12, C07C 211/63, C07F 9/54

(54) **METHOD FOR PRODUCING HETEROPOLYOXOMETALATE HAVING MODIFIED LACUNARY SITE, OR MIXTURE THEREOF**

(30) Priority: 31.10.2023 JP 2023187315
(71) Applicant: TOKYO OHKA KOGYO CO., LTD., Kawasaki-shi, Kanagawa 211 0012 (JP)
(72) Inventor: NISHIZAWA, Akito, Kawasaki-shi, Kanagawa 211-0012 (JP); INARI, Takatoshi, Kawasaki-shi, Kanagawa 211-0012 (JP)
(74) Representative: Plasseraud IP
(86) International application number: PCT/JP2024/038006
(87) International publication number: WO 2025/094828

(57) **Abstract**

To provide a novel method for producing a heteropolyacid salt having a modified lacunary site or a mixture thereof, including a step of converting a Keggin-type or Dawson-type heteropolyacid salt into a mono-lacunary Keggin-type or Dawson-type heteropolyacid salt, and a step of reacting the mono-lacunary Keggin-type or Dawson-type heteropolyacid salt with a P, Si, Ge, or Sn compound having one or more hydro groups or organic groups and two or more leaving groups to obtain a heteropolyacid salt having a modified lacunary site.

## Description

### Technical Field

The present invention relates to a method for producing a heteropolyacid salt having a modified lacunary site or a mixture thereof.

### Background Art

The polyacid is an anionic metal oxide cluster represented by the general formula [MₓO_{y}]ⁿ⁻ (wherein x, y, and n all denote natural numbers). The metal atom M constituting the polyacid is referred to as a polyatom and is, for example, Mo (hexavalent or pentavalent), W (hexavalent or pentavalent), V (pentavalent), Nb (pentavalent), Ta (pentavalent), or the like. The polyacid can be broadly divided into an isopolyacid composed of the polyatom M and an oxoacid, and a heteropolyacid ([X_{w}MₓO_{y}]ⁿ⁻ (wherein w, x, y, and n all denote natural numbers)) containing a different type of heteroatom X (for example, P⁵⁺, Si⁴⁺, Ge⁴⁺, B³⁺ or the like as a heteroatom X) in addition to the polyatom M and oxygen.

Furthermore, it is known that there are a plurality of lacunary species having a defect in part of the basic skeleton in the polyacid. A terminal oxygen atom at a lacunary site having a defect in part of the basic skeleton of the polyacid has a certain degree of negative charge density on the oxygen atom and has high reactivity and nucleophilicity. Thus, various functional polyacid complexes or organic/inorganic hybrid structures can be formed by reacting the terminal oxygen atom with an electrophilic atom or molecule.

Patent Literature 1 discloses, as a method for producing a metal-substituted polyoxometalate, a method including a step of reacting a polyoxometalate having one or more lacunary sites with a metal complex having a central metal and an organic ligand in a reaction liquid containing the polyoxometalate and the metal complex to produce a metal-substituted polyoxometalate.

### Citation List

### Patent Literature

PTL 1: U.S. Patent No. 11420871

### Summary of Invention

### Technical Problem

An object of the present invention is to provide a novel method for producing a heteropolyacid salt having a modified lacunary site or a mixture thereof.

As a result of extensive studies to solve the above problems, the present inventors have completed the present invention by finding that a novel method for producing a heteropolyacid salt having a modified lacunary site or a mixture thereof can be provided by using a production method including a step of converting a Keggin-type or Dawson-type heteropolyacid salt into a mono-lacunary Keggin-type or Dawson-type heteropolyacid salt, and a step of reacting the mono-lacunary Keggin-type or Dawson-type heteropolyacid salt with a P, Si, Ge, or Sn compound having one or more hydro groups or organic groups and two or more leaving groups to obtain a heteropolyacid salt having a modified lacunary site.

More specifically, the present invention relates to the following inventions.
<1> A method for producing a heteropolyacid salt having a modified lacunary site or a mixture thereof, including (1) a step of converting a Keggin-type or Dawson-type heteropolyacid salt into a mono-lacunary Keggin-type or Dawson-type heteropolyacid salt, and
   (2) a step of reacting the mono-lacunary Keggin-type or Dawson-type heteropolyacid salt with a P, Si, Ge, or Sn compound having one or more hydro groups or organic groups and two or more leaving groups to produce a heteropolyacid salt having a modified lacunary site represented by the general formula (I) or a mixture thereof.

   (A^{p+})_{q}[C^{(pxq)-}] (I)

   [In the formula (I),
   A^{p+} each independently denotes H⁺, a metal ion, or an ammonium ion,
   C^{(pxq)-} denotes a mono-lacunary Keggin-type or Dawson-type heteropolyacid anion, the heteropolyacid anion having a modified lacunary site, and
   p denotes an integer in the range of 1 to 5, and q denotes a real number]
<2> The method for producing a heteropolyacid salt having a modified lacunary site or a mixture thereof according to <1>, wherein the step (1) includes
   (1a) a step of adjusting a pH of a solution containing the Keggin-type or Dawson-type heteropolyacid salt to 5.0 or less.
<3> The method for producing a heteropolyacid salt having a modified lacunary site or a mixture thereof according to <1>, wherein the step (1) includes
   (1a) a step of adjusting a pH of a solution containing the Keggin-type or Dawson-type heteropolyacid salt to 5.0 or less, and
   (1b) a step of adjusting a pH of the solution to a range of 4 to 8 using a weak base or a base.
<4> The method for producing a heteropolyacid salt having a modified lacunary site or a mixture thereof according to <1>, wherein the step (2) includes
   (2a) a step of reacting the mono-lacunary Keggin-type or Dawson-type heteropolyacid salt with a P, Si, Ge, or Sn compound having one or more hydro groups or organic groups and two or more leaving groups in a solution with a pH of 5.0 or less.
<5> The method for producing a heteropolyacid salt having a modified lacunary site or a mixture thereof according to <1>, wherein the Keggin-type or Dawson-type heteropolyacid salt includes Mo, W, V, Nb, or Ta as a polyatom and P, Si, B, S, or Ge as a heteroatom.
<6> The method for producing a heteropolyacid salt having a modified lacunary site or a mixture thereof according to <1>, wherein the mono-lacunary Keggin-type or Dawson-type heteropolyacid salt is represented by the general formula (III-1) or (III-2).

   [XM₁₁O₃₉]^{c21-} (III-1)

   [X₂M₁₇O₆₁]^{c22-} (III-2)

   [wherein
   X denotes a heteroatom of P, Si, B, S, or Ge,
   M denotes a polyatom of Mo, W, V, Nb, or Ta, and
   c21 and c22 are natural numbers]
<7> The method for producing a heteropolyacid salt having a modified lacunary site or a mixture thereof according to <1>, wherein the organic group has one or more halogen atoms, haloalkyl groups, hydroxy groups, thiol groups, nitro groups, cyano groups, carboxy groups, amino groups, sulfo groups, epoxy groups, glycidyl groups, or amide groups.
<8> The method for producing a heteropolyacid salt having a modified lacunary site or a mixture thereof according to <1>, wherein the organic group has one or more ethylenically unsaturated double bonds.
<9> The method for producing a heteropolyacid salt having a modified lacunary site or a mixture thereof according to <1>, wherein the organic group has one or more hydroxy groups or carboxy groups, each having a protective group.
<10> The method for producing a heteropolyacid salt having a modified lacunary site or a mixture thereof according to <1>, wherein C^{(pxq)-} is represented by any one of the general formulae (VI-1) to (VI-8).

   [XM₁₁O₃₉(R^{1A}X')₂O]^{c31-} (VI-1)

   [XM₁₁O₃₉(R^{1B1}R^{1B2}Si)₂]^{c31-} (VI-2)

   [XM₁₁O₃₉(R^{1C}P=O)₂]^{c31-} (VI-3)

   [XM₁₁O₃₉(R^{1D}X'')]^{c31-} (VI-4)

   [X₂M₁₇O₆₁(R^{1A}X')₂O]^{c32-} (VI-5)

   [X₂M₁₇O₆₁(R^{1B1}R^{1B2}Si)₂]^{c32-} (VI-6)

   [X₂M₁₇O₆₁(R^{1C}P=O)₂]^{c32-} (VI-7)

   [X₂M₁₇O₆₁(R^{1D}X'')]^{c32-} (VI-8)

   [wherein
   X denotes a heteroatom of P, Si, B, S, or Ge,
   M denotes a polyatom of Mo, W, V, Nb, or Ta, and
   X' denotes a heteroatom of Si or Ge,
   X" denotes a heteroatom of Si, Ge, or Sn,
   R^{1A} to R^{1D} each independently denote a hydro group, an optionally substituted C₁₋₁₈ hydrocarbyl group, an optionally substituted 3- to 18-membered non-aromatic heterocyclic group, or an optionally substituted 5- to 18-membered aromatic heterocyclic group,
   a divalent carbon atom at any position of R^{1A} to R^{1D} except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
   a hydrogen atom in R^{1A} to R^{1D} may be substituted with, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group, and
   c31 and c32 are natural numbers]
<11> The method for producing a heteropolyacid salt having a modified lacunary site or a mixture thereof according to <1>, further including (3) a step of salt-exchanging the heteropolyacid salt having the modified lacunary site represented by the general formula (I) or a mixture thereof.
<12> The method for producing a heteropolyacid salt having a modified lacunary site or a mixture thereof according to <11>, wherein the step (3) includes (3a) a step of salt-exchanging the heteropolyacid salt having the modified lacunary site represented by the general formula (I) or a mixture thereof with an alkali metal salt, an alkaline-earth metal salt, an aluminum salt, or an onium salt.
<13> The method for producing a heteropolyacid salt having a modified lacunary site or a mixture thereof according to <12>, wherein the onium salt is an organic sulfonium salt, an organic iodonium salt, an organic ammonium salt, or an organic phosphonium salt.
<14> The method for producing a heteropolyacid salt having a modified lacunary site or a mixture thereof according to <11>, wherein the step (3) includes (3b) a step of obtaining a heteropolyacid salt having a modified lacunary site represented by the general formula (VII) or a mixture thereof.

   (A^{m+})ₐ(Bⁿ⁺)_{b}(C^{(am+bn)-}) (VII)

   [wherein
   when b is 0,
   A^{m+} each independently denotes H⁺, a metal ion, or an ammonium ion, at least one of which is an alkali metal ion, an alkaline-earth metal ion, or Al³⁺, different from A^{p+},
   when b is a real number greater than 0,
   A^{m+} each independently denotes H⁺, a metal ion, or an ammonium ion,
   Bⁿ⁺ each independently denotes an organic sulfonium cation, an organic sulfonium dication, an organic iodonium cation, an organic ammonium cation, an organic ammonium dication, an organic phosphonium cation, or an organic phosphonium dication,
   C^{(am+bn)-} denotes a mono-lacunary Keggin-type or Dawson-type heteropolyacid anion, the heteropolyacid anion having a modified lacunary site, and
   m denotes an integer in the range of 1 to 5, n denotes an integer of 1 or 2, and a is a real number]
<15> The method for producing a heteropolyacid salt having a modified lacunary site represented by the general formula (VII) or a mixture thereof, including a step of substituting a cation moiety of the heteropolyacid salt having the modified lacunary site represented by the general formula (I) or a mixture thereof with a metal ion, an ammonium ion, or an organic ammonium cation by a salt exchange reaction and a step of performing salt exchange with an onium salt.

   (A^{m+})ₐ(Bⁿ⁺)_{b}(C^{(am+bn)-}) (VII)

   [wherein
   A^{m+} each independently denotes H⁺, a metal ion, or an ammonium ion,
   Bⁿ⁺ each independently denotes an organic sulfonium cation, an organic sulfonium dication, an organic iodonium cation, an organic ammonium cation, an organic ammonium dication, an organic phosphonium cation, or an organic phosphonium dication,
   C^{(am+bn)-} denotes a mono-lacunary Keggin-type or Dawson-type heteropolyacid anion, the heteropolyacid anion having a modified lacunary site, and
   m denotes an integer in the range of 1 to 5, n denotes an integer of 1 or 2, a denotes a real number, and b denotes a real number greater than 0]
<16> A method for producing a heteropolyacid salt having a modified lacunary site or a mixture thereof, the method including a step of washing a heteropolyacid salt having a modified lacunary site represented by the general formula (VII) or a mixture thereof with an acidic aqueous solution with a pH of 6 or less.

   (A^{m+})ₐ(Bⁿ⁺)_{b}(C^{(am+bn)-}) (VII)

   [wherein
   A^{m+} each independently denotes H⁺, a metal ion, or an ammonium ion,
   Bⁿ⁺ each independently denotes an organic sulfonium cation, an organic sulfonium dication, an organic iodonium cation, an organic ammonium cation, an organic ammonium dication, an organic phosphonium cation, or an organic phosphonium dication,
   C^{(am+bn)-} denotes a mono-lacunary Keggin-type or Dawson-type heteropolyacid anion, the heteropolyacid anion having a modified lacunary site, and
   m denotes an integer in the range of 1 to 5, n denotes an integer of 1 or 2, a denotes a real number, and b denotes a real number greater than 0]
<17> The method for producing a heteropolyacid salt having a modified lacunary site or a mixture thereof according to <16>, wherein the step of washing with an acidic aqueous solution with a pH of 6 or less is a step of reducing the content of each metal or metal ion selected from Li, Na, Mg, Al, K, Ca, Ti, V, Cr, Mn, Fe, Co, Ni, Cu, Zn, As, Sr, Zr, Mo, Ag, Cd, Sn, Sb, Ba, W, Pb, and Au to less than 100 ppb in the heteropolyacid salt having the modified lacunary site represented by the general formula (VII) or a mixture thereof, excluding the metals or metal ions contained in the polyatom, the heteroatom, and A^{m+} of the heteropolyacid salt having the modified lacunary site represented by the general formula (VII) or a mixture thereof.

### Advantageous Effects of Invention

The present invention can provide a novel method for producing a heteropolyacid salt having a modified lacunary site or a mixture thereof. A heteropolyacid salt having a modified lacunary site or a mixture thereof with fewer impurities can be obtained by a production method according to the present invention.

### Description of Embodiments

Preferred embodiments of the present invention will be described in detail below. However, the present invention is not limited to the following embodiments.

In the present description, the term "(meth)acryloyl group" is used to mean both an acryloyl group and a methacryloyl group.

The term "halogen atom", as used herein, refers to a fluorine atom, a chlorine atom, a bromine atom, or an iodine atom.

In the present description, for example, the term "C₁₋₆" or the like means the number of carbon atoms of a group serving as a mother nucleus.

The term "C₁₋₁₈ hydrocarbylene group", as used herein, refers to a divalent hydrocarbon group formed by removing two hydrogen atoms from a hydrocarbon with 1 to 18 carbon atoms. The hydrocarbylene group may be linear, branched, or partially or fully cyclic. Examples of the hydrocarbylene group include an alkylene group and an arylene group.

A divalent carbon atom at any position of the "C₁₋₁₈ hydrocarbylene group" may be replaced by -O-, -S-, -C(=O)-, -COO-, -OCO-, -CONH-, -NHCO-, -NH(C=O)O-, -S-, -SO-, or -SO₂- (provided that adjacent divalent carbon atoms are not replaced at the same time).

The "C₁₋₁₈ hydrocarbylene group" is, for example, but not limited to, a "C₁₋₁₈ alkylene group", such as a methylene group, an ethylene group, a n-propylene group, an i-propylene group, a cyclopentadiyl group, a cyclohexanediyl group, an oxyethane-1,1-diyl group, an oxyethane-1,2-diyl group, an oxypropane-1,3-diyl group, an oxypropane-1,2-diyl group, a 2-methylpropane-1,3-diyl group, or an oxyethyleneoxyethane-1,1-diyl group; a "C₆₋₁₈ arylene group", such as a 1,4-phenylene group, a 1,3-phenylene group, a 1,2-phenylene group, a 1,4-naphthylene group, a 1,5-naphthylene group, a 1,8-naphthylene group, a 4,4'-biphenylene group, an anthracenediyl group, a phenanthrenediyl group, a naphthacenediyl group, a pyrenediyl group, a perylenediyl group, or a chrysenediyl group; or the like.

The term "C₁₋₁₈ alkyl group", as used herein, refers to a linear or branched alkyl group with 1 to 18 carbon atoms.

Furthermore, a divalent carbon atom at any position excluding the terminals contained in the "C₁₋₁₈ alkyl group" may be replaced by -O-, -C(=O)-, -C(=O)O-, -OCO-, - CONH-, -NHCO-, -NH(C=O)O-, -S-, or -SO₂-. However, adjacent divalent carbon atoms are not replaced at the same time.

The "C₁₋₁₈ alkyl group" is, for example, but not limited to, a methyl group, an ethyl group, a n-propyl group, an i-propyl group, a n-butyl group, an i-butyl group, a sec-butyl group, a t-butyl group, a n-pentyl group, an i-pentyl group, a sec-pentyl group, a t-pentyl group, a neopentyl group, a 1-methylbutyl group, a 2-methylbutyl group, a 1,1-dimethylpropyl group, a 1,2-dimethylpropyl group, a n-hexyl group, an i-hexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, a n-heptyl group, a n-octyl group, a n-nonyl group, a n-decyl group, a n-undecyl group, a n-dodecyl group, or the like.

The "C₁₋₁₈ alkyl group" in which a divalent carbon atom at any position excluding the terminals thereof is replaced by -O-, -C(=O)-, -C(=O)O-, -OCO-, -CONH-, -NHCO-, - NH(C=O)O-, -S-, or -SO₂- is, for example, but not limited to, a 2-methoxyethoxymethyl group, an ethoxycarbonylmethyl group, or the like.

The term "C₁₋₁₈ haloalkyl group", as used herein, refers to a group in which one or more hydrogen atoms of the "C₁₋₁₈ alkyl group" are substituted by a halogen atom.

The "C₁₋₁₈ haloalkyl group" is, for example, but not limited to, a dichloromethyl group, a trifluoromethyl group, a 2,2-difluoroethyl group, a 2,2,2-trifluoroethyl group, a pentafluoroethyl group, a 3,3,3-trifluoropropyl group, or the like.

The term "C₂₋₁₈ alkenyl group", as used herein, refers to an alkenyl group with 2 or more carbon atoms having one or more double bonds in the "C₁₋₁₈ alkyl group" and includes an alkadienyl group, an alkatrienyl group, and the like.

The "C₂₋₁₈ alkenyl group" is, for example, but not limited to, a vinyl group (ethenyl group), an allyl group (2-propenyl group), a 1-propenyl group, an isopropenyl group (1-methylvinyl group), a 1-butenyl group, a 2-butenyl group, a 3-butenyl group, a pentenyl group, a hexenyl group, a heptenyl group, an octenyl group, a nonenyl group, a decenyl group, an undecenyl group, a dodecenyl group, or the like.

The term "C₂₋₁₈ alkynyl group", as used herein, refers to an alkynyl group with 2 or more carbon atoms having one or more triple bonds in the "C₁₋₁₈ alkyl group".

The "C₂₋₁₈ alkynyl group" is, for example, but not limited to, an ethynyl group, a 1-propynyl group, a 2-propynyl group, a pentynyl group, a hexynyl group, a heptynyl group, an octynyl group, a nonynyl group, a decynyl group, an undecynyl group, a dodecynyl group, or the like.

The term "C₃₋₁₈ alicyclic group", as used herein, refers to a hydrocarbon group with 3 to 18 carbon atoms and with a monocyclic or polycyclic structure in whole or in part. The alicyclic group includes a cycloalkyl group, a cycloalkenyl group, a cycloalkynyl group, a monocycloalkyl group, a polycycloalkyl group, and the like.

A divalent carbon atom at any position excluding the terminals contained in the "C₃₋₁₈ alicyclic group" may be replaced by -O-, -C(=O)-, -C(=O)O-, -OCO-, -CONH-, -NHCO-, -NH(C=O)O-, -S-, or -SO₂- (provided that adjacent divalent carbon atoms are not replaced at the same time).

The "C₃₋₁₈ cycloalkyl group" is, for example, but not limited to, a cyclopropyl group, a cyclobutyl group, a cyclopentyl group, a 1-i-propylcyclopentane-1-yl group, a cyclohexyl group, a t-butylcyclohexyl group, a tricyclodecanyl group, a cycloheptyl group, a cyclooctyl group, a cyclodecyl group, a 2-methyladamantane-2-yl group, a 2-i-propyladamantane-2-yl group, a bornyl group, a norbornyl group, a fenchyl group, a pinanyl group, an adamantyl group, a tricyclodecyl group, a tetracyclododecyl group, a cyclopropylmethyl group, a cyclobutylmethyl group, a cyclopentylmethyl group, a cyclohexylmethyl group, a bornylmethyl group, a norbornylmethyl group, an adamantylmethyl group, a 1-methylcyclopentyloxycarbonylmethyl group, a cyclopentenyl group, a cyclohexenyl group, a cycloheptenyl group, or the like.

The term "3- to 18-membered non-aromatic heterocyclic group", as used herein, refers to a 3- to 18-membered non-aromatic heterocyclic group containing one or more heteroatoms selected from the group consisting of a nitrogen atom, an oxygen atom, and a sulfur atom, may be monocyclic, polycyclic, or condensed, and may be saturated or partially unsaturated.

The "3- to 18-membered non-aromatic heterocyclic group" is, for example, but not limited to, an aziridinyl group, an azetidil group, a pyrrolidinyl group, a pyrrolyl group, a piperidinyl group, a piperazinyl group, a morpholinyl group, a thiomorpholinyl group, a tetrahydrofuryl group, a tetrahydropyranyl group, an oxetanyl group, a tetrahydrofuryl group, a tetrahydropyranyl group, an imidazolinyl group, an oxazolinyl group, a 2,5-diazabicyclo[2.2.1]heptyl group, a 2,5-diazabicyclo[2.2.2]octyl group, a 3,8-diazabicyclo[3.2.1]octyl group, a 1,4-diazabicyclo[4.3.0]nonyl group, a 1-azaadamantyl group, a 2-azaadamantyl group, or the like.

The term "C₂₋₁₈ aryl group", as used herein, refers to an aromatic hydrocarbon ring group with 6 to 18 carbon atoms or an aromatic heterocyclic group with 2 to 10 carbon atoms; in the case of an aromatic heterocyclic group, 2 to 10 carbon atoms and one or more heteroatoms selected from the group consisting of a nitrogen atom, an oxygen atom, and a sulfur atom form a ring, and the ring may be monocyclic, polycyclic, or a fused ring.

The "C₂₋₁₈ aryl group" is, for example, but not limited to, a phenyl group, a 1-naphthyl group, a 2-naphthyl group, an azulenyl group, a pentalenyl group, a heptalenyl group, an indacenyl group, an acenaphthyl group, a phenanthrenyl group, an anthracenyl group, or the like.

The term "C₇₋₁₈ aralkyl group", as used herein, refers to a group in which a substitutable portion of a "C₁₋₁₂ alkyl group" is substituted by the "C₂₋₁₂ aryl group".

The "C₇₋₁₈ aralkyl group" is, for example, but not limited to, a benzyl group, a phenethyl group, a 3-phenylpropyl group, a 4-phenylbutyl group, a 1-naphthylmethyl group, or a 2-naphthylmethyl group, or the like.

The term "C₁₋₁₈ hydrocarbyl group", as used herein, refers to a monovalent group formed by removing one hydrogen atom from a hydrocarbon with 1 to 18 carbon atoms. Examples of the hydrocarbyl group include an alkyl group, an alkenyl group, an alkynyl group, an alicyclic group, an aryl group, an aralkyl group, and the like.

A divalent carbon atom at any position excluding the terminals contained in the "C₁₋₁₈ hydrocarbyl group" may be replaced by -O-, -C(=O)-, -C(=O)O-, -OCO-, -CONH-, - NHCO-, -NH(C=O)O-, -S-, or -SO₂- (provided that adjacent divalent carbon atoms are not replaced at the same time). The same applies to the "C₁₋₁₈ hydrocarbyl group" and the like used in the following description of the definition of the "C₁₋₁₈ hydrocarbyloxy group" and the like.

The "C₁₋₁₈ hydrocarbyl group" is, for example, but not limited to, a "C₁₋₁₈ alkyl group", a "C₂₋₁₈ alkenyl group", a "C₂₋₁₈ alkynyl group", a "C₃₋₁₈ alicyclic group", a "C₂₋₁₈ aryl group", a "C₇₋₁₈ aralkyl group", or the like.

The term "C₁₋₁₈ hydrocarbyloxy group", as used herein, refers to a group in which an oxygen atom (-O-) is bonded to the "C₁₋₁₈ hydrocarbyl group".

The "C₁₋₁₈ hydrocarbyloxy group" is, for example, but not limited to, a "C₁₋₁₈ alkoxy group", such as a methoxy group, an ethoxy group, a n-propoxy group, an i-propoxy group, a n-butoxy group, an i-butoxy group, a sec-butoxy group, a t-butoxy group, a n-pentoxy group, an i-pentoxy group, a sec-pentoxy group, a n-hexoxy group, an i-hexoxy group, a 1,1-dimethylpropyloxy group, a 1,2-dimethylpropyloxy group, a 2,2-dimethylpropyloxy group, a 1-methyl-2-ethylpropyloxy group, a 1-ethyl-2-methylpropyloxy group, a 1,1,2-trimethylpropyloxy group, a 1,2,2-trimethylpropyloxy group, a 1,1-dimethylbutyloxy group, a 1,2-dimethylbutyloxy group, a 2,2-dimethylbutyloxy group, a 2,3-dimethylbutyloxy group, a 1,3-dimethylbutyloxy group, a 2-ethylbutyloxy group, a 2-methylpentyloxy group, or a 3-methylpentyloxy group; a "C₃₋₁₈ alicyclic oxy group", such as a cyclopropyloxy group, a cyclobutyloxy group, a cyclopentyloxy group, a cyclohexyloxy group, a cycloheptyloxy group, a cyclooctyloxy group, a 1-methylcyclopentyloxycarbonylmethoxy group, a 1-ethylcyclohexyloxycarbonylmethoxy group, or a 1-methyladamantyloxycarbonylmethoxy group; a "C₆₋₁₈ aryloxy group", such as a phenyloxy group, a 1-naphthyloxy group, a 2-naphthyloxy group, an azulenyloxy group, a pentalenyloxy group, a heptalenyloxy group, an indacenyloxy group, an acenaphthyloxy group, a phenanthrenyloxy group, or an anthracenyloxy group; or the like.

In the "C₁₋₁₈ hydrocarbyloxy group", a divalent carbon atom at any position excluding the terminals in the "C₁₋₁₈ hydrocarbyl group" is preferably replaced by -O-, - C(=O)-, and/or -C(=O)O-, the "C₁₋₁₈ hydrocarbyloxy group" is more preferably a "C₁₋₁₈ hydrocarbyloxycarbonylalkyloxy group", and from the perspective of solubility, a carbon atom bonded to the oxygen atom of the hydrocarbyloxy is still more preferably a tertiary carbon. Specific examples of the hydrocarbyloxy include optionally substituted ethylcyclopentyloxy, methyladamantyloxy, ethyladamantyloxy, t-butyloxy, and the like.

The term "C₁₋₁₈ hydrocarbylcarbonyl group", as used herein, refers to a group in which a carbonyl group (-C(=O)-) is bonded to the "C₁₋₁₈ hydrocarbyl group".

The "C₁₋₁₈ hydrocarbylcarbonyl group" is, for example, but not limited to, a "C₁₋₁₈ alkyl carbonyl group", such as an acetyl group, a propionyl group, an isopropionyl group, a butyryl group, an isobutyryl group, a valeryl group, an isovaleryl group, a pentanoyl group, a 3-methylbutanoyl group, a pivaloyl group, a hexanoyl group, or a heptanoyl group; a "C₃₋₁₈ alicyclic carbonyl group", such as a cyclopropyl carbonyl group, a cyclobutyl carbonyl group, a cyclopentyl carbonyl group, a 2-methylcyclopentyl carbonyl group, a 3-methylcyclopentyl carbonyl group, a cyclohexyl carbonyl group, a 2-methylcyclohexyl carbonyl group, a 3-methylcyclohexyl carbonyl group, a 4-methylcyclohexyl carbonyl group, or an adamantyl carbonyl group; a "C₆₋₁₈ aryl carbonyl group", such as a benzoyl group, a 1-naphthoyl group, or a 2-naphthoyl group; or the like.

The term "C₁₋₁₈ hydrocarbylcarbonyloxy group", as used herein, refers to a group in which an oxygen atom (-O-) is bonded to the "C₁₋₁₈ hydrocarbylcarbonyl group".

The "C₁₋₁₈ hydrocarbylcarbonyloxy group" is, for example, but not limited to, a "C₁₋₁₈ alkyl carbonyloxy group", such as a methyl carbonyloxy group, an ethyl carbonyloxy group, a n-propyl carbonyloxy group, an isopropyl carbonyloxy group, a n-butyl carbonyloxy group, an isobutyl carbonyloxy group, a t-butyl carbonyloxy group, a n-pentyl carbonyloxy group, an isopentyl carbonyloxy group, or a hexyl carbonyloxy group; a "C₃₋₁₈ alicyclic carbonyloxy group", such as a cyclopropyl carbonyloxy group, a cyclobutyl carbonyloxy group, a cyclopentyl carbonyloxy group, or a cyclohexyl carbonyloxy group; a "C₆₋₁₈ aryl carbonyloxy group", such as a phenyl carbonyloxy group, a naphthyl carbonyloxy group, an acenaphthyl carbonyloxy group, a phenanthrenyl carbonyloxy group, or an anthracenyl carbonyloxy group; or the like.

The term "C₁₋₁₈ hydrocarbyloxycarbonyl group", as used herein, refers to a group in which a carbonyl group (-C(=O)-) is bonded to a "C₁₋₁₈ hydrocarbyloxy group".

The "C₁₋₁₈ hydrocarbyloxycarbonyl group" is, for example, but not limited to, a "C₁₋₁₈ alkoxycarbonyl group", such as a methoxycarbonyl group, an ethoxycarbonyl group, a n-propoxycarbonyl group, an i-propoxycarbonyl group, a n-butoxycarbonyl group, an i-butoxycarbonyl group, a sec-butoxycarbonyl group, a t-butoxycarbonyl group, a n-pentoxycarbonyl group, or a neopentyloxycarbonyl group; a "C₃₋₁₈ alicyclic oxycarbonyl group", such as a cyclopropyloxycarbonyl group, a cyclobutyloxycarbonyl group, a cyclopentyloxycarbonyl group, a cyclohexyloxycarbonyl group, a 2-methylcyclopentyloxycarbonyl group, a 3-methylcyclopentyloxycarbonyl group, a 2-methylcyclohexyloxycarbonyl group, a 3-methylcyclohexyloxycarbonyl group, or a 4-methylcyclohexyloxycarbonyl group; a "C₆₋₁₈ aryloxycarbonyl group", such as a phenoxycarbonyl group, a naphthoxycarbonyl group, an acenaphthyloxycarbonyl group, a phenanthrenyloxycarbonyl group, or an anthracenyloxycarbonyl group; or the like.

The term "C₁₋₁₈ hydrocarbyloxycarbonyloxy group", as used herein, refers to a group in which an oxygen atom (-O-) is bonded to the "C₁₋₁₈ hydrocarbyloxycarbonyl group".

The "C₁₋₁₈ hydrocarbyloxycarbonyloxy group" is, for example, but not limited to, a "C₁₋₁₈ alkoxycarbonyloxy group", such as a methoxycarbonyloxy group, an ethoxycarbonyloxy group, a n-propyloxycarbonyloxy group, an i-propyloxycarbonyloxy group, a n-butoxycarbonyloxy group, an i-butoxycarbonyloxy group, a sec-butoxycarbonyloxy group, a t-butoxycarbonyloxy group, a n-pentyloxycarbonyloxy group, an i-pentyloxycarbonyloxy group, or a n-hexyloxycarbonyloxy group; a "C₃₋₁₈ alicyclic oxycarbonyloxy group", such as a cyclopropyloxycarbonyloxy group, a cyclobutyloxycarbonyloxy group, a cyclopentyloxycarbonyloxy group, or a cyclohexyloxycarbonyloxy group; a "C₆₋₁₈ aryloxycarbonyloxy group", such as a phenoxycarbonyloxy group, a naphthoxycarbonyloxy group, an acenaphthyloxycarbonyloxy group, a phenanthrenyloxycarbonyloxy group, or an anthracenyloxycarbonyloxy group; or the like.

The term "C₁₋₁₈ hydrocarbylamino group", as used herein, refers to a group in which one "C₁₋₁₈ hydrocarbyl group" is bonded to an amino group.

The "C₁₋₁₈ hydrocarbylamino group" is, for example, but not limited to, a "C₁₋₁₈ alkylamino group", such as a methylamino group, an ethylamino group, a n-propylamino group, an i-propylamino group, a n-butylamino group, an i-butylamino group, a sec-butylamino group, a t-butylamino group, a n-pentylamino group, an i-pentylamino group, a neopentylamino group, or a n-hexylamino group; a "C₃₋₁₈ alicyclic amino group", such as a cyclopropylamino group, a cyclobutylamino group, a cyclopentylamino group, a 2-methylcyclopentylamino group, a 3-methylcyclopentylamino group, a cyclohexylamino group, a 2-methylcyclohexylamino group, a 3-methylcyclohexylamino group, or a 4-methylcyclohexylamino group; a "C₆₋₁₈ arylamino group", such as a phenylamino group, a 1-naphthylamino group, or a 2-naphthylamino group; or the like.

The term "di-C₁₋₁₈ hydrocarbylamino group", as used herein, refers to a group in which two identical or different "C₁₋₁₈ hydrocarbyl groups" are bonded to an amino group.

The "di-C₁₋₁₈ hydrocarbylamino group" is, for example, but not limited to, a "di-C₁₋₁₈ alkylamino group", such as a dimethylamino group, a diethylamino group, a di-n-propylamino group, a diisopropylamino group, a di-n-butylamino group, a diisobutylamino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, or an N-ethyl-N-n-hexylamino group; a "di-C₃₋₁₈ alicyclic amino group", such as a dicyclopropylamino group, a dicyclobutylamino group, a dicyclopentylamino group, or a dicyclohexylamino group; a "di-C₆₋₁₈ arylamino group", such as a diphenylamino group or a phenylnaphthylamino group; an "N-C₁₋₁₈ alkyl-N-C₃₋₁₈ cycloalkylamino group", such as an N-methylcyclopentaneamino group or an N-methylcyclohexylamino group; an "N-C₁₋₁₈ alkyl-N-C₆₋₁₈ arylamino group", such as an N-methyl-2-phenylethylamino group or an N-ethyl-N-(4-methylphenyl)amino group; or the like.

The term "C₁₋₁₈ hydrocarbylaminocarbonyl group", as used herein, refers to a group in which a carbonyl group (-C(=O)-) is bonded to the "C₁₋₁₈ hydrocarbylamino group".

The "C₁₋₁₈ hydrocarbylaminocarbonyl group" is, for example, but not limited to, a "C₁₋₁₈ alkylaminocarbonyl group", such as a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butylaminocarbonyl group, a n-pentylaminocarbonyl group, a 2-pentylaminocarbonyl group, a neopentylaminocarbonyl group, a 4-methyl-2-pentylaminocarbonyl group, a n-hexylaminocarbonyl group, or a 3-methyl-n-pentylaminocarbonyl group; a "C₃₋₁₈ alicyclic aminocarbonyl group", such as a cyclopropylaminocarbonyl group, a cyclobutylaminocarbonyl group, a cyclopentylaminocarbonyl group, a cyclohexylaminocarbonyl group, a 2-methylcyclopentylaminocarbonyl group, a 3-methylcyclopentylaminocarbonyl group, a 2-methylcyclohexylaminocarbonyl group, a 3-methylcyclohexylaminocarbonyl group, or a 4-methylcyclohexylaminocarbonyl group; or a "C₆₋₁₈ arylaminocarbonyl group", such as a phenylaminocarbonyl group, a 1-naphthylaminocarbonyl group, or a 2-naphthylaminocarbonyl group.

The term "di-C₁₋₁₈ hydrocarbylaminocarbonyl group", as used herein, refers to a group in which a carbonyl group (-C(=O)-) is bonded to a "di-C₁₋₁₈ hydrocarbylamino group".

The "di-C₁₋₁₈ hydrocarbylaminocarbonyl group" is, for example, but not limited to, a "di-C₁₋₁₈ alkylamino group", such as a dimethylaminocarbonyl group, a diethylaminocarbonyl group, a di-n-propylaminocarbonyl group, a diisopropylaminocarbonyl group, a di-n-butylaminocarbonyl group, a diisobutylaminocarbonyl group, a di-t-butylaminocarbonyl group, a di-n-pentylaminocarbonyl group, a di-n-hexylaminocarbonyl group, an N-ethyl-N-methylaminocarbonyl group, an N-methyl-N-n-propylaminocarbonyl group, an N-isopropyl-N-methylaminocarbonyl group, an N-n-butyl-N-methylaminocarbonyl group, an N-isobutyl-N-methylaminocarbonyl group, an N-t-butyl-N-methylaminocarbonyl group, an N-methyl-N-n-pentylaminocarbonyl group, an N-n-hexyl-N-methylaminocarbonyl group, an N-ethyl-N-n-propylaminocarbonyl group, an N-ethyl-N-isopropylaminocarbonyl group, an N-n-butyl-N-ethylaminocarbonyl group, an N-ethyl-N-isobutylaminocarbonyl group, an N-t-butyl-N-ethylaminocarbonyl group, an N-ethyl-N-n-pentylaminocarbonyl group, or an N-ethyl-N-n-hexylaminocarbonyl group; a "di-C₃₋₁₈ alicyclic aminocarbonyl group", such as a dicyclopropylaminocarbonyl group, a dicyclobutylaminocarbonyl group, a dicyclopentylaminocarbonyl group, or a dicyclohexylaminocarbonyl group; a "di-C₆₋₁₈ arylaminocarbonyl group", such as a diphenylaminocarbonyl group or a phenylnaphthylaminocarbonyl group; or the like.

The term "C₁₋₁₈ hydrocarbylcarbonylamino group", as used herein, refers to a group in which an amino group is bonded to the "C₁₋₁₈ hydrocarbylcarbonyl group".

The "C₁₋₁₈ hydrocarbylcarbonylamino group" is, for example, but not limited to, a "C₁₋₁₈ alkyl carbonylamino group", such as a methyl carbonylamino group, an ethyl carbonylamino group, a n-propyl carbonylamino group, an i-propyl carbonylamino group, a n-butyl carbonylamino group, an i-butyl carbonylamino group, a sec-butyl carbonylamino group, a t-butyl carbonylamino group, a n-pentyl carbonylamino group, an i-pentyl carbonylamino group, or a n-hexyl carbonylamino group; a "C₃₋₁₈ alicyclic carbonylamino group", such as a cyclopropyl carbonylamino group, a cyclobutyl carbonylamino group, a cyclopentyl carbonylamino group, or a cyclohexyl carbonylamino group; a "C₆₋₁₈ aryl carbonylamino group", such as a phenyl carbonylamino group, a naphthyl carbonylamino group, an acenaphthyl carbonylamino group, a phenanthrenyl carbonylamino group, or an anthracenyl carbonylamino group; or the like.

The term "C₁₋₁₈ hydrocarbylaminocarbonyloxy group", as used herein, refers to a group in which an oxygen atom (-O-) is bonded to the "C₁₋₁₈ hydrocarbylaminocarbonyl group".

The "C₁₋₁₈ hydrocarbylaminocarbonyloxy group" is, for example, but not limited to, a "C₁₋₁₈ alkylaminocarbonyloxy group", such as a methylaminocarbonyloxy group, an ethylaminocarbonyloxy group, or a n-propylaminocarbonyloxy group; a "C₃₋₁₈ alicyclic aminocarbonyloxy group", such as a cyclopropylaminocarbonyloxy group or a cyclohexylaminocarbonyloxy group; a "C₆₋₁₈ arylaminocarbonyloxy group", such as a phenylaminocarbonyloxy group or a 1-naphthylaminocarbonyloxy group; or the like.

The term "di-C₁₋₁₈ hydrocarbylaminocarbonyloxy group", as used herein, refers to a group in which an oxygen atom (-O-) is bonded to the "di-C₁₋₁₈ hydrocarbylaminocarbonyl group".

The "di-C₁₋₁₈ hydrocarbylaminocarbonyloxy group" is, for example, but not limited to, a "di-C₁₋₁₈ alkylaminocarbonyloxy group", such as a dimethylaminocarbonyloxy group, a diethylaminocarbonyloxy group, or a di-n-propylaminocarbonyloxy group; or the like.

The term "C₁₋₁₈ hydrocarbylaminocarbonylamino group", as used herein, refers to a group in which the "C₁₋₁₈ hydrocarbylaminocarbonyl group" is bonded to an amino group.

The "C₁₋₁₈ hydrocarbylaminocarbonylamino group" is, for example, but not limited to, a "C₁₋₁₈ alkylaminocarbonylamino group", such as a methylaminocarbonylamino group, an ethylaminocarbonyloxy group, or a n-propylaminocarbonylamino group; a "C₃₋₁₈ alicyclic aminocarbonylamino group", such as a cyclopropylaminocarbonylamino group or a cyclohexylaminocarbonylamino group; a "C₆₋₁₈ arylaminocarbonylamino group", such as a phenylaminocarbonylamino group or a 1-naphthylaminocarbonylamino group; or the like.

The term "di-C₁₋₁₈ hydrocarbylaminocarbonylamino group", as used herein, refers to a group in which a "di-C₁₋₁₈ hydrocarbylaminocarbonyl group" is bonded to an amino group.

The "di-C₁₋₁₈ hydrocarbylaminocarbonylamino group" is, for example, but not limited to, a "di-C₁₋₁₈ alkylaminocarbonylamino group", such as a dimethylaminocarbonylamino group, a diethylaminocarbonylamino group, or a di-n-propylaminocarbonylamino group; or the like.

The term "C₁₋₁₈ hydrocarbyloxycarbonylamino group", as used herein, refers to a group in which the "C₁₋₁₈ hydrocarbyloxycarbonyl group" is bonded to an amino group.

The "C₁₋₁₈ hydrocarbyloxycarbonylamino group" is, for example, but not limited to, a "C₁₋₁₈ alkoxycarbonylamino group", such as a methoxycarbonylamino group, an ethoxycarbonylamino group, a n-propoxycarbonylamino group, an i-propoxycarbonylamino group, a n-butoxycarbonylamino group, or a t-butoxycarbonylamino group; a "C₃₋₁₈ alicyclic oxycarbonylamino group", such as a cyclopropyloxycarbonylamino group or a cyclohexyloxycarbonylamino group; a "C₆₋₁₈ aryloxycarbonylamino group", such as a phenyloxycarbonylamino group or a 1-naphthyloxycarbonylamino group; or the like.

The term "C₁₋₁₈ hydrocarbylthio group", as used herein, refers to a group in which a sulfur atom (-S-) is bonded to the "C₁₋₁₈ hydrocarbyl group".

The "C₁₋₁₈ hydrocarbylthio group" is, for example, but not limited to, a "C₁₋₁₈ alkylthio group", such as a methylthio group, an ethylthio group, a n-propylthio group, an i-propylthio group, a n-butylthio group, an i-butylthio group, a t-butylthio group, a n-pentylthio group, or a n-hexylthio group; a "C₃₋₁₈ alicyclic thio group", such as a cyclopropylthio group, a cyclobutylthio group, a cyclopentylthio group, a cyclohexylthio group, a 2-methylcyclopentylthio group, a 3-methylcyclopentylthio group, a 2-methylcyclohexylthio group, a 3-methylcyclohexylthio group, or a 4-methylcyclohexylthio group; a "C₆₋₁₈ arylthio group", such as a phenylthio group, a 1-naphthylthio group, a 2-naphthylthio group, an acenaphthylthio group, a phenanthrenylthio group, or an anthracenylthio group; or the like.

The term "C₁₋₁₈ hydrocarbylsulfinyl group", as used herein, refers to a group in which a sulfinyl group (-S(=O)-) is bonded to the "C₁₋₁₈ hydrocarbyl group".

The "C₁₋₁₈ hydrocarbylsulfinyl group" is, for example, but not limited to, a "C₁₋₁₈ alkylsulfinyl group", such as a methylsulfinyl group, an ethylsulfinyl group, a n-propylsulfinyl group, an i-propylsulfinyl group, a n-butylsulfinyl group, a t-butylsulfinyl group, a pentylsulfinyl group, or a hexylsulfinyl group; a "C₃₋₁₈ alicyclic sulfinyl group", such as a cyclopropylsulfinyl group, a cyclobutylsulfinyl group, a cyclopentylsulfinyl group, a cyclohexylsulfinyl group, a 2-methylcyclopentylsulfinyl group, a 3-methylcyclopentylsulfinyl group, a 2-methylcyclohexylsulfinyl group, a 3-methylcyclohexylsulfinyl group, or a 4-methylcyclohexylsulfinyl group; a "C₆₋₁₈ arylsulfinyl group", such as a phenylsulfinyl group, a naphthylsulfinyl group, an acenaphthylsulfinyl group, a phenanthrenylsulfinyl group, or an anthracenylsulfinyl group; or the like.

The term "C₁₋₁₈ hydrocarbylsulfonyl group", as used herein, refers to a group in which a sulfonyl group (-SO₂-) is bonded to the "C₁₋₁₈ hydrocarbyl group".

The "C₁₋₁₈ hydrocarbylsulfonyl group" is, for example, but not limited to, a "C₁₋₁₈ alkylsulfonyl group", such as a methylsulfonyl group, an ethylsulfonyl group, a n-propylsulfonyl group, an i-propylsulfonyl group, a n-butylsulfonyl group, a t-butylsulfonyl group, or a pentylsulfonyl group; a "C₃₋₁₈ alicyclic sulfonyl group", such as a cyclopropylsulfonyl group, a cyclobutylsulfonyl group, a cyclopentylsulfonyl group, a cyclohexylsulfonyl group, a 2-methylcyclopentylsulfonyl group, a 3-methylcyclopentylsulfonyl group, a 2-methylcyclohexylsulfonyl group, a 3-methylcyclohexylsulfonyl group, or a 4-methylcyclohexyl group; a "C₆₋₁₈ arylsulfonyl group", such as a phenylsulfonyl group, a naphthylsulfonyl group, an acenaphthylsulfonyl group, a phenanthrenylsulfonyl group, or an anthracenylsulfonyl group; or the like.

The term "acid-dissociable group", as used herein, refers to a group that substitutes for a hydrogen atom of a hydroxy group (including a phenolic hydroxy group and the like) or a carboxy group and that is dissociated by the action of an acid.

The acid-dissociable group is, for example, an acid-dissociable group G for a hydroxy group or a carboxy group represented by the following general formula (G-1) or (G-2).

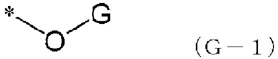

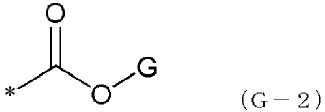

The acid-dissociable group G is not particularly limited, provided that it can be dissociated by the action of an acid, and a known and commonly used group can be used. The acid-dissociable group G is, for example, a "tertiary-carbon-type acid-dissociable group G_{A}" represented by the following general formula (g-1), or the like.

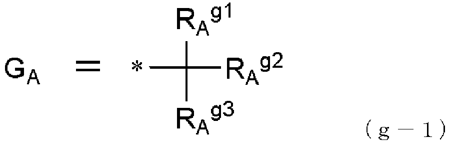

### <Tertiary-carbon-type Acid-Dissociable Group G_{A}>

As the acid-dissociable group G, an acid-dissociable group in which a carbon that is directly bonded to a polar group and to which R_{A}^{g1} to R_{A}^{g3} are bonded is a tertiary carbon atom, such as a "tertiary-carbon-type acid-dissociable group G_{A}" represented by the following general formula (g-1), can be used.

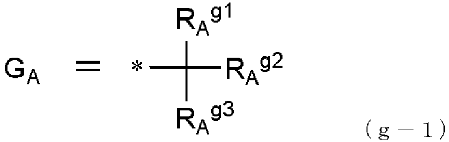

"R_{A}^{g1}" denotes an optionally substituted C₁₋₁₂ hydrocarbyl group in which any divalent carbon atom except the terminal positions may be replaced by -O-, -C(=O)-, - C(=O)O-, -OCO-, -CONH-, -NHCO-, -S-, or -SO₂- (provided that adjacent divalent carbon atoms are not replaced at the same time).

R_{A}^{g1} preferably denotes a C₁₋₁₂ alkyl group, a C₃₋₁₂ alicyclic group, a C₆₋₁₂ aryl group, or a C₇₋₁₂ aralkyl group each optionally having a substituent and any divalent carbon atom except the terminal positions may be replaced by -O-, -C(=O)-, -C(=O)O-, -OCO-, -CONH-, -NHCO-, -S-, or -SO₂- (provided that adjacent divalent carbon atoms are not replaced at the same time),
more preferably a C₁₋₁₂ alkyl group or a C₃₋₁₂ alicyclic group each optionally having a substituent and any divalent carbon atom except the terminal positions may be replaced by -O-, -C(=O)-, -C(=O)O-, -OCO-, -CONH-, -NHCO-, -S-, or -SO₂- (provided that adjacent divalent carbon atoms are not replaced at the same time).

"R_{A}^{g2}" and "R_{A}^{g3}" each independently denote an optionally substituted C₁₋₁₂ hydrocarbyl group, and a divalent carbon atom at any position except the terminal positions may be substituted with -O-, -C(=O)-, -C(=O)O-, -OCO-, -CONH-, -NHCO-, -S-, or -SO₂-(provided that adjacent divalent carbon atoms are not substituted at the same time), or R_{A}^{g2} and R_{A}^{g3} may be combined to form a C₃₋₁₈ alicyclic group or a 3- to 18-membered non-aromatic heterocyclic group, each optionally having a substituent, and a divalent carbon atom at any position except the terminal positions may be substituted with -O-, -C(=O)-, - C(=O)O-, -OCO-, -CONH-, -NHCO-, -S-, or -SO₂- (provided that adjacent divalent carbon atoms are not substituted at the same time).

In R_{A}^{g2} and R_{A}^{g3}, preferably, R_{A}^{g2} and R_{A}^{g3} may be combined to form a C₃₋₁₈ alicyclic group or a 3- to 18-membered non-aromatic heterocyclic group, each optionally having a substituent, and a divalent carbon atom at any position except the terminal positions may be substituted with -O-, -C(=O)-, -C(=O)O-, -OCO-, -CONH-, -NHCO-, -S-, or -SO₂-(provided that adjacent divalent carbon atoms are not substituted at the same time).

More preferably, the substituted C₃₋₁₈ alicyclic group or the 3- to 18-membered non-aromatic heterocyclic group, each optionally having a substituent, has a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, and a divalent carbon atom at any position except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

A tertiary-carbon-type acid-dissociable group G_{A} represented by the general formula (g-1) is, for example, but not limited to, a t-butyl group, a t-amyl group, a 1,1-dimethylpropyl group, a 1-methyl-1-cyclopentyl group, a 1-ethyl-1-cyclopentyl group, a 1-methyl-1-cyclohexyl group, a 1-ethyl-1-cyclohexyl group, a 2-methyl-2-adamantyl group, a 2-ethyl-2-adamantyl group, a 1-(1-methoxy-2-methylpropan-2-yl)cyclopentyl group, a 1-(1-ethoxy-2-methylpropan-2-yl)cyclopentyl group, or the like.

### <<Tertiary-Carbon-Type Acid-Dissociable Group G_{A1} and G_{A2}>>

In the "tertiary-carbon-type acid-dissociable group G_{A}" represented by the general formula (g-1), the case where R_{A}^{g2} and R_{A}^{g3} may be combined to form a C₃₋₁₈ alicyclic group or a 3- to 18-membered non-aromatic heterocyclic group, each optionally having a substituent, is, for example, a tertiary-carbon-type acid-dissociable group G_{A1} represented by the following general formula (g-1-1), a tertiary-carbon-type acid-dissociable group G_{A2} represented by the following general formula (g-1-2), or the like.

### <<Tertiary-Carbon-Type Acid-Dissociable Group G_{A1} Represented by General Formula (g-1-1)>>

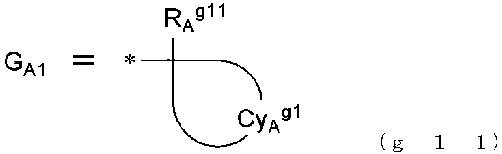

In the tertiary-carbon-type acid-dissociable group G_{A1} represented by the general formula (g-1-1), R_{A}^{g11} denotes a C₁₋₁₂ alkyl group, a C₃₋₁₂ alicyclic group, a C₆₋₁₂ aryl group, or a C₇₋₁₂ aralkyl group, each optionally having a substituent, and any divalent carbon atom except the terminal positions may be substituted with -O-, -C(=O)-, -C(=O)O-, -OCO-, - CONH-, -NHCO-, -S-, or -SO₂- (provided that adjacent divalent carbon atoms are not substituted at the same time).

Cy_{A}^{g1}, together with the tertiary carbon atom, forms a C₃₋₁₈ alicyclic group or a 3- to 18-membered non-aromatic heterocyclic group, each optionally having a substituent, and any divalent carbon atom may be substituted with -O-, -C(=O)-, -C(=O)O-, -OCO-, -CONH-, -NHCO-, -S-, or -SO₂- (provided that adjacent divalent carbon atoms are not substituted at the same time).

Preferably, Cy_{A}^{g1}, together with the tertiary carbon atom, forms a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, each optionally having a substituent, and a divalent carbon atom at any position except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

The tertiary-carbon-type acid-dissociable group G_{A1} represented by the general formula (g-1-1) is, for example, the following tertiary-carbon-type acid-dissociable group.

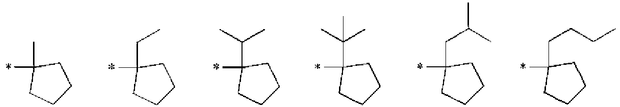

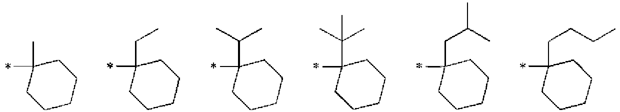

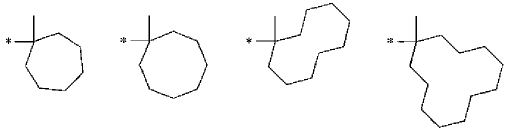

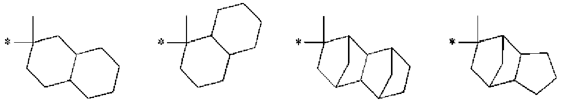

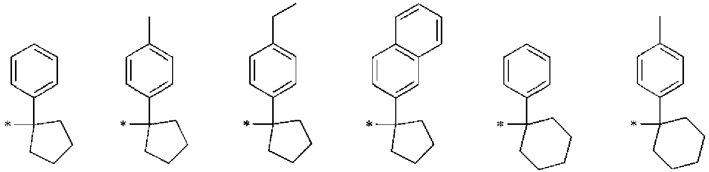

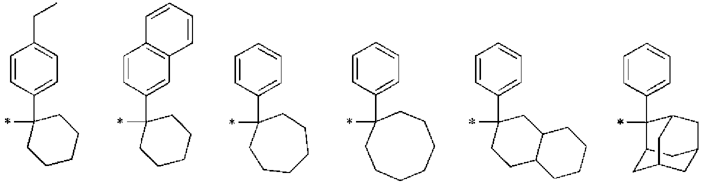

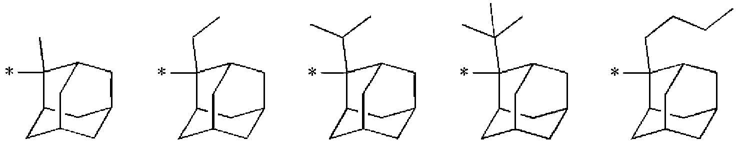

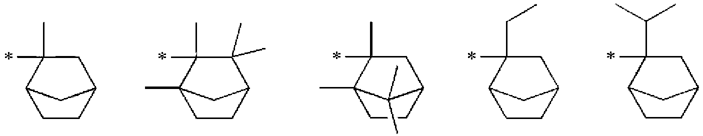

### <<Tertiary-Carbon-Type Acid-Dissociable Group G_{A2} Represented by General Formula (g-1-2)>>

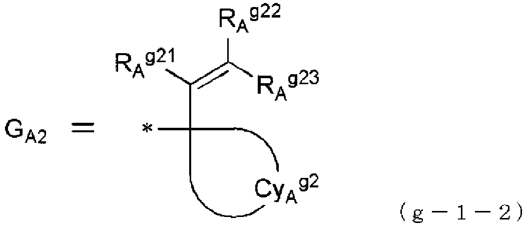

In the general formula (g-1-2), R_{A}^{g21} to R_{A}^{g23} each independently denote a hydro group or an optionally substituted C₁₋₁₂ hydrocarbyl group, any divalent carbon atom except the terminal positions may be substituted with -O-, -C(=O)-, -C(=O)O-, -OCO-, -CONH-, - NHCO-, -S-, or -SO₂- (provided that adjacent divalent carbon atoms are not substituted at the same time), and R_{A}^{g21} and R_{A}^{g22}, and/or R_{A}^{g22} and R_{A}^{g23} may be directly bonded to each other by a single bond or may be bonded to each other via a divalent linker such as, -O-, - S-, -C(=O)-, -S(=O)-, -S(=O)₂-, -C(=O)O-, or a C₁₋₃ alkylene group, to form a ring.

The case where R_{A}^{g21} and R_{A}^{g22}, and/or R_{A}^{g22} and R_{A}^{g23} form a ring together with a carbon atom contained in the ethylenically unsaturated double bond is, for example, a case where they form a cyclopentenyl group, a cyclohexenyl group, a cyclopentylidene ethenyl group, a cyclohexylidene ethenyl group, or the like, each optionally having a substituent.

Cy_{A}^{g2}, together with the tertiary carbon atom, forms a C₃₋₁₈ alicyclic group or a 3- to 18-membered non-aromatic heterocyclic group, each optionally having a substituent, and any divalent carbon atom may be substituted with -O-, -C(=O)-, -C(=O)O-, -OCO-, -CONH-, -NHCO-, -S-, or -SO₂- (provided that adjacent divalent carbon atoms are not substituted at the same time).

Preferably, Cy_{A}^{g2}, together with the tertiary carbon atom, forms a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, each optionally having a substituent, and a divalent carbon atom at any position except the terminal positions may be substituted with -O-, -C(=O)-, -C(=O)O-, -OCO-, -CONH-, - NHCO-, -S-, or -SO₂- (provided that adjacent divalent carbon atoms are not substituted at the same time).

The tertiary-carbon-type acid-dissociable group G_{A2} represented by the general formula (g-1-2) is, for example, the following tertiary-carbon-type acid-dissociable group.

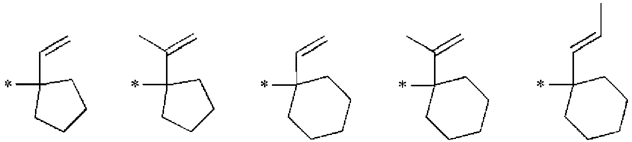

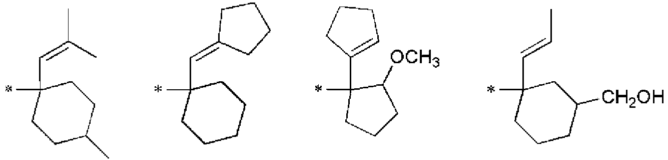

The phrase "a hydroxy group or a carboxy group each having a protective group", as used herein, refers to a hydroxy group (including a phenolic hydroxy group) or a carboxy group in which the hydroxy group or the carboxy group is protected by an ether protective group, a silyl ether protective group, an acetal protective group, an acyl protective group, an oxycarbonyl (alkyl) protective group, a dihydrocarbylaminocarbonyl protective group, or the like, or refers to a hydroxy group (including a phenolic hydroxy group) or a carboxy group each having an acid-dissociable group.

The ether protective group is, for example, but not limited to, a methyl group, a benzyl group, a p-methoxybenzyl group, a t-butyl group, a triphenylmethyl group, a p-methoxyphenyldiphenylmethyl group, a di(p-methoxyphenyl)phenylmethyl group, or the like.

The silyl ether protective group is, for example, but not limited to, a t-butyldimethylsilyl group (TBS), a triisopropylsilyl group (TIPS), a trimethylsilyl group (TMS), a triethylsilyl group (TES), a t-butyldiphenylsilyl group (TBDPS), or the like.

The acetal protective group is, for example, but not limited to, a methoxymethyl group, an ethoxyethyl group, a 2-tetrahydropyranyl group (THP), a methoxyethoxymethyl group, or the like.

The acyl protective group is, for example, but not limited to, an acetyl group, a pivaloyl group, a benzoyl group, or the like.

The oxycarbonyl (alkyl) protective group is, for example, but not limited to, a t-butoxycarbonyl group or the like.

The dihydrocarbylaminocarbonyl protective group is, for example, but not limited to, a dimethylaminocarbonyl group, a diethylaminocarbonyl group, a diisopropylaminocarbonyl group, an N-phenyl-N-methyl-aminocarbonyl group, or the like.

The protective group is, for example, a protective group P for a hydroxy group or a carboxy group represented by the following general formula (P-1) or (P-2).

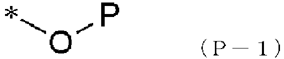

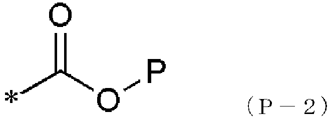

The protective group P is not particularly limited and may be a known and commonly used protective group. The protective group P other than the "acid-dissociable group" is, for example, an "acetal protective group P_{A}" represented by the following general formula (p-1), a "silyl ether protective group P_{B}" represented by the following general formula (p-2), an "aminocarbonyl protective group P_{C}" represented by the following general formula (p-3), or the like. Each of them will be sequentially described below.

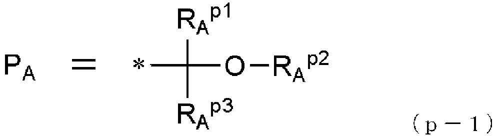

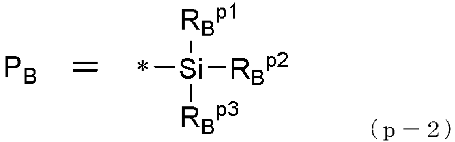

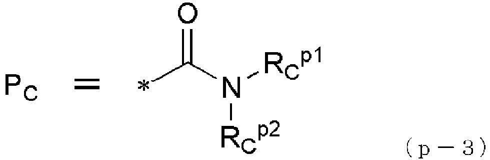

### <Acetal Protective Group P_{A}>

The protective group P may be a protective group in which an oxygen atom is bonded to a carbon atom directly bonded to an oxygen atom of a hydroxy group, a phenolic hydroxy group, or a carboxy group, such as the "acetal protective group P_{A}" represented by the following general formula (p-1).

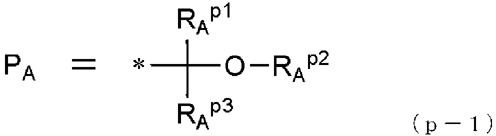

"R_{A}^{p1}" and "R_{A}^{p3}" each independently denote a hydro group or an optionally substituted C₁₋₁₂ hydrocarbyl group, and any divalent carbon atom except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

"R_{A}^{p2}" denotes an optionally substituted C₁₋₁₂ hydrocarbyl group, and any divalent carbon atom except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

Preferably, R_{A}^{p1} and R_{A}^{p3} denote a hydro group, or a C₁₋₁₂ alkyl group or a C₃₋₁₂ alicyclic group, the alkyl group and the alicyclic group each optionally having a substituent, and any divalent carbon atom except the terminal positions may be substituted with -O-, - C(=O)-, -C(=O)O-, -OCO-, -CONH-, -NHCO-, -S-, or -SO₂- (provided that adjacent divalent carbon atoms are not substituted at the same time),
more preferably a hydro group, or a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

Preferably, R_{A}^{p2} denotes a C₁₋₁₂ alkyl group or a C₃₋₁₂ alicyclic group, the alkyl group and the alicyclic group each optionally having a substituent, and any divalent carbon atom except the terminal positions may be substituted with -O-, -C(=O)-, -C(=O)O-, -OCO-, - CONH-, -NHCO-, -S-, or -SO₂- (provided that adjacent divalent carbon atoms are not substituted at the same time),
more preferably a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, - NHCO-, -S-, or -SO₂- (provided that adjacent divalent carbon atoms are not substituted at the same time).

The acetal protective group P_{A} represented by the general formula (p-1) is, for example, a methoxymethoxy group, an ethoxymethoxy group, a n-propoxymethoxy group, a n-butoxymethoxy group, a 2,2-dimethylpropoxymethoxy group, a 2,2-dimethylbutoxymethoxy group, a cyclohexyloxymethoxy group, a 1-ethoxyethoxy group, a 1-n-butoxyethoxy group, a 1-cyclohexyloxyethoxy group, or the like.

### <Silyl Ether Protective Group P_{B}>

The protective group P may be a protective group in which a silicon atom is directly bonded to an oxygen atom of a hydroxy group, a phenolic hydroxy group, or a carboxy group, such as a "silyl ether protective group P_{B}" represented by the following general formula (p-2).

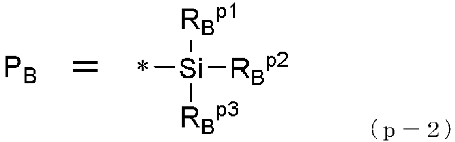

"R_{B}^{p1}" to "R_{B}^{p3}" each independently denote an optionally substituted C₁₋₁₂ hydrocarbyl group in which any divalent carbon atom except the terminal positions may be replaced by -O-, -C(=O)-, -C(=O)O-, -OCO-, -CONH-, -NHCO-, -S-, or -SO₂- (provided that adjacent divalent carbon atoms are not replaced at the same time).

R_{B}^{p1} to R_{B}^{p3} preferably denote a C₁₋₁₂ alkyl group or a C₃₋₁₂ alicyclic group each optionally having a substituent and any divalent carbon atom except the terminal positions may be replaced by -O-, -C(=O)-, -C(=O)O-, -OCO-, -CONH-, -NHCO-, -S-, or -SO₂-(provided that adjacent divalent carbon atoms are not replaced at the same time), more preferably a C₁₋₆ alkyl group or a C₃₋₈ alicyclic group each optionally having a substituent and any divalent carbon atom except the terminal positions may be replaced by -O-, -C(=O)-, -C(=O)O-, -OCO-, -CONH-, -NHCO-, -S-, or -SO₂- (provided that adjacent divalent carbon atoms are not replaced at the same time).

The silyl ether protective group P_{B} represented by the general formula (p-2) is, for example, but not limited to, a t-butyldimethylsilyl group (TBS), a triisopropylsilyl group (TIPS), a trimethylsilyl group (TMS), a triethylsilyl group (TES), a t-butyldiphenylsilyl group (TBDPS), or the like.

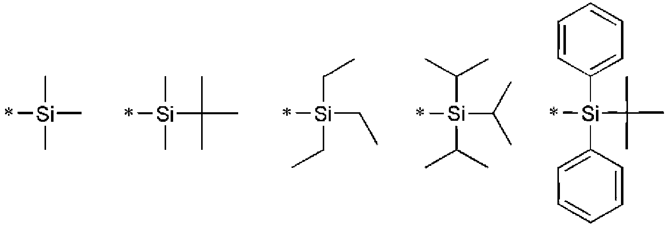

### <Aminocarbonyl Protective Group P_{C}>

The protective group P may be a protective group in which an aminocarbonyl group is bonded to an oxygen atom of a hydroxy group, a phenolic hydroxy group, or a carboxy group, such as an "aminocarbonyl protective group P_{C}" represented by the following general formula (p-3).

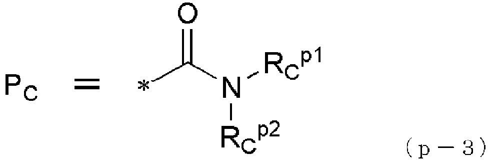

"R_{C}^{p1}" and "R_{C}^{p2}" each independently denote a hydro group, or an optionally substituted C₁₋₁₂ hydrocarbyl group in which any divalent carbon atom except the terminal positions may be replaced by -O-, -C(=O)-, -C(=O)O-, -OCO-, -CONH-, -NHCO-, -S-, or - SO₂- (provided that adjacent divalent carbon atoms are not replaced at the same time).

Preferably, R_{C}^{p1} and R_{C}^{p2} denote a hydro group, or a C₁₋₁₂ alkyl group or a C₃₋₁₂ alicyclic group, the alkyl group and the alicyclic group each optionally having a substituent, and any divalent carbon atom except the terminal positions may be substituted with -O-, - C(=O)-, -C(=O)O-, -OCO-, -CONH-, -NHCO-, -S-, or -SO₂- (provided that adjacent divalent carbon atoms are not substituted at the same time),
more preferably a hydro group, or a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

The aminocarbonyl protective group P_{C} represented by the general formula (p-3) is, for example, but not limited to, a dimethylaminocarbonyl group, a diethylaminocarbonyl group, a diisopropylaminocarbonyl group, an N-phenyl-N-methyl-aminocarbonyl group, or the like.

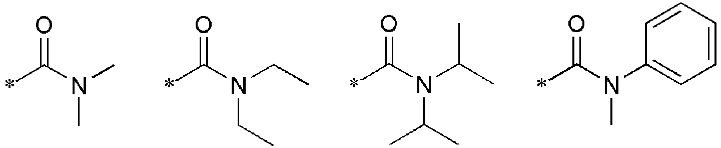

The phrase "optionally substituted" or "optionally having a substituent", as used herein, is not particularly limited provided that it is chemically acceptable and has the advantages of the present invention.

The "substituent" is, for example, (1) a halogen atom, (2) a haloalkyl group, (3) a hydroxy group, (4) a thiol group, (5) a nitro group, (6) a cyano group, (7) a carboxy group, (8) an amino group, (9) a sulfo group, (10) a vinyl group, (11) an allyl group, (12) a (meth)acryloyl group, (13) a (meth)acryloyloxy group, (14) a (meth)acrylamide group, (15) a styryl group, (16) an epoxy group, (17) a glycidyl group, (18) an amide group, (19) a hydroxy group or a carboxy group each having a protective group, or (20) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of hydrogen atoms may be substituted with (1) to (19), and a divalent carbon atom at any position of these substituents except the terminal positions may be substituted with -O-, - C(=O)-, -C(=O)O-, -OCO-, -CONH-, -NHCO-, -NH(C=O)O-, -S-, or -SO₂- (provided that adjacent divalent carbon atoms are not substituted at the same time), or the like.

### [1. Step (1)]

The method for producing a heteropolyacid salt having a modified lacunary site or a mixture thereof according to the present embodiment includes, as a step (1), (1) a step of converting a Keggin-type or Dawson-type heteropolyacid salt into a mono-lacunary Keggin-type or Dawson-type heteropolyacid salt or a mixture thereof.

The step (1) may include, as a step (1a), a step of adjusting the pH of a solution containing the Keggin-type or Dawson-type heteropolyacid salt to 5 or less.

The step (1) may also include, after the step (1a), as a step (1b), a step of adjusting the pH of the solution to a range of 4 to 8 using a weak base or a base.

### <Keggin-Type or Dawson-Type Heteropolyacid Salt>

The heteropolyacid anion, which is the anion moiety of the Keggin-type or Dawson-type heteropolyacid salt, may include Mo, W, V, Nb, or Ta as a polyatom and P, Si, B, S, or Ge as a heteroatom.

The Keggin-type or Dawson-type heteropolyacid anion may be a Keggin-type or Dawson-type heteropolyacid anion represented by the general formula (II-1) or (II-2).

[XM₁₂O₄₀]^{c11-} (II-1)

[X₂M₁₈O₆₂]^{c12-} (II-2)

[In the formula (II-1) and (II-2),
X denotes a heteroatom of P, Si, B, S, or Ge,
M denotes a polyatom of Mo, W, V, Nb, or Ta, and
c11- and c12- denote the number of negative charges, and c11 and c12 are natural numbers]

The Keggin-type heteropolyacid anion is, for example, [PW₁₂O₄₀]³⁻, [SiW₁₂O₄₀]⁴⁻, [BW₁₂O₄₀]⁵⁻, [SW₁₂O₄₀]²⁻, [GeW₁₂O₄₀]⁴⁻, or the like. The Dawson-type heteropolyacid anion is, for example, [P₂W₁₈O₆₂]⁶⁻, [Si₂W₁₈O₆₂]⁸⁻, [S₂W₁₈O₆₂]⁴⁻, or the like.

It should be noted that the Keggin-type or Dawson-type heteropolyacid anion may include all isomers, such as geometric isomers, optical isomers, rotational isomers, stereoisomers, and tautomers, which are structurally present, and isomer mixtures, and is not limited to the description of convenient chemical formulae.

The counter cation that is the cation moiety of the Keggin-type or Dawson-type heteropolyacid salt may be, for example, but is not limited to, H⁺, Li⁺, Na⁺, K⁺, Rb⁺, Cs⁺, Be²⁺, Mg²⁺, Ca²⁺, Sr²⁺, Ba²⁺, Al³⁺, Co³⁺, Bi³⁺, Zr⁴⁺, Hf⁴⁺, Bi⁵⁺, NH⁴⁺, a quaternary ammonium cation, or a combination thereof.

### <Mono-lacunary Keggin-Type or Dawson-Type Heteropolyacid Salt>

The heteropolyacid anion, which is the anion moiety of the mono-lacunary Keggin-type or Dawson-type heteropolyacid salt, may include Mo, W, V, Nb, or Ta as a polyatom and P, Si, B, S, or Ge as a heteroatom.

The mono-lacunary Keggin-type or Dawson-type heteropolyacid anion may be a Keggin-type or Dawson-type heteropolyacid anion represented by the general formula (III-1) or (III-2).

[XM₁₁O₃₉]^{c21-} (III-1)

[X₂M₁₇O₆₁]^{c22-} (III-2)

[In the formula (III-1) and (III-2),
X denotes a heteroatom of P, Si, B, S, or Ge,
M denotes a polyatom of Mo, W, V, Nb, or Ta, and
c21- and c22- denote the number of negative charges, and c21 and c22 are natural numbers]

The mono-lacunary Keggin-type heteropolyacid anion is, for example, [PMo₁₁O₃₉]⁷⁻, [PW₁₁O₃₉]⁷⁻, [SiMo₁₁O₃₉]⁸⁻, [SiW₁₁O₃₉]⁸⁻, [BMo₁₁O₃₉]⁹⁻, [BW₁₁O₃₉]⁹⁻, [SMo₁₁O₃₉]⁶⁻, [SW₁₁O₃₉]⁶⁻, [BMo₁₁O₃₉]⁹⁻, [BW₁₁O₃₉]⁹⁻, [GeMo₁₁O₃₉]⁸⁻, [GeW₁₁O₃₉]⁸⁻, or the like. The mono-lacunary Dawson-type heteropolyacid anion is, for example, [P₂W₁₇O₆₁]¹⁰⁻, [S₂W₁₇O₆₁]⁸⁻, or the like.

It should be noted that the mono-lacunary Keggin-type or Dawson-type heteropolyacid salt may include all isomers, such as geometric isomers, optical isomers, rotational isomers, stereoisomers, and tautomers, which are structurally present, and isomer mixtures, and is not limited to the description of convenient chemical formulae.

The counter cation that is the cation moiety of the mono-lacunary Keggin-type or Dawson-type heteropolyacid salt may be, for example, but is not limited to, H⁺, Li⁺, Na⁺, K⁺, Rb⁺, Cs⁺, Be²⁺, Mg²⁺, Ca²⁺, Sr²⁺, Ba²⁺, Al³⁺, Co³⁺, Bi³⁺, Zr⁴⁺, Hf⁴⁺, Bi⁵⁺, NH⁴⁺, a quaternary ammonium cation, or a combination thereof.

### [1-1. Step (1a)]

The step (1) may include, as a step (1a), a step of adjusting the pH of a solution containing the Keggin-type or Dawson-type heteropolyacid salt to 5 or less.

When the step (1) includes the step (1a), the amount of impurities in the finally obtained heteropolyacid salt having a modified lacunary site or a mixture thereof is smaller than that in the case of using a known and commonly used method described below. Thus, the step (1) preferably includes the step (1a).

The solution in the step (1a) is, for example, but not limited to, a solution containing water and/or one or more organic solvents. Examples of the organic solvents include methanol, ethanol, propanol, butanol, ethylene glycol, propylene glycol, glycerol, acetone, ethyl acetate, dichloroethane, chloroform, acetone, tetrahydrofuran, acetonitrile, dimethyl sulfoxide, dimethylformamide, and the like.

The pH of the solution in the step (1a) is preferably 5.0 or less, preferably 4.8 or less, more preferably -1 to 4.5, still more preferably -0.5 to 4.0.

The method for adjusting the pH of the solution to 5.0 or less in the step (1a) is, for example, but is not limited to, a method of adding an acidic solution, or the like.

The type of acid contained in the acidic solution is, for example, an inorganic acid, such as hydrochloric acid, sulfuric acid, nitric acid, sulfamic acid, or phosphoric acid; an organic acid having a carboxy group, such as tartaric acid, oxalic acid, formic acid, acetic acid, propionic acid, malic acid, lactic acid, citric acid, succinic acid, maleic acid, fumaric acid, glycolic acid, trifluoroacetic acid, or trichloroacetic acid; an organic acid having a sulfo group, such as methanesulfonic acid, ethanesulfonic acid, sulfosuccinic acid, m-toluenesulfonic acid, or p-toluenesulfonic acid; or the like. These acids may be used alone or in combination of two or more types thereof.

The acid is preferably hydrochloric acid, phosphoric acid, lactic acid, or acetic acid, more preferably hydrochloric acid or acetic acid, depending on the type of the solution and the like.

In the step (1a), after the pH of the solution is adjusted to 5.0 or less, the solution may be heated to raise the temperature for the reaction. The temperature of the solution is preferably 15°C or more, 25°C or more, or 35°C or more, and is preferably 80°C or less, 70°C or less, or 60°C or less.

On the other hand, a known and commonly used method for producing a mono-lacunary Keggin-type or Dawson-type heteropolyacid salt may be a method for reacting a solution containing a salt of an oxide containing a polyatom of a heteropolyacid and a salt of an oxide containing a heteroatom of a heteropolyacid by adjusting the pH of the solution to a range of approximately 5 to 7 using 4 M aqueous hydrochloric acid or the like, and then adjusting the pH to a range of 6 to 7 to produce a mono-lacunary Keggin-type or Dawson-type heteropolyacid salt. The pH of the salt of the oxide containing the polyatom of the heteropolyacid, which is usually basic, is adjusted to the range of approximately 5 to 7 using 4 M aqueous hydrochloric acid or the like.

The salt of the oxide containing the polyatom of the heteropolyacid is, for example, a molybdate, such as Na₂MoO₄, K₂MoO₄, Rb₂MoO₄, Cs₂MoO₄, CaMoO₄, MgMoO₄, SrMoO₄, or BaMoO₄; a tungstate, such as Na₂WO₄, K₂WO₄, Rb₂WO₄, Cs₂WO₄, CaWO₄, MgWO₄, SrWO₄, or BaWO₄; a vanadate, such as NH₄VO₃, Na₃VO₄, or NaVO₃; a niobate, such as NaNbO₃, BaNb₂O₆, or SrNb₂O₆; a tantalate, such as KTaO₃, NaTaO₃, or SrTa₂O₆; or the like.

The salt of the oxide containing the heteroatom of the heteropolyacid salt is, for example, a phosphate, such as Li₃PO₄, Na₃PO₄, K₃PO₄, or Rb₃PO₄; a silicate, such as Li₂SiO₃, Na₂SiO₃, MgSiO₃, CaSiO₃, FeSiO₃, MnSiO₃, or K₂SiO₃; a germanate, such as Na₂GeO₃, BaGeO₃, Ca₂GeO₄, or Sr₂GeO₄; a borate, such as LiBO₂, NaBO₂, KBO₂, RbBO₂, MgB₂O₄, SrB₂O₄, BaB₂O₄, CoB₂O₄, or MnB₂O₄; or the like.

However, when a heteropolyacid salt having a modified lacunary site or a mixture thereof is produced by the known and commonly used method, a heteropolyacid salt having a modified lacunary site or a mixture thereof containing a large amount of impurities is obtained. Thus, it is necessary to adopt a method other than the known and commonly used method as a method for producing a heteropolyacid salt having a modified lacunary site or a mixture thereof.

### [1-2. Step (1b)]

The step (1) may include, after the step (1a), as a step (1b), a step of adjusting the pH of the solution to a range of 4 to 8 using a weak base or a base.

For the solution, similarly to the solution exemplified in the step (1a), water and/or one or more organic solvents can be used.

The weak base or base is, for example, but not limited to, sodium carbonate, potassium carbonate, sodium hydrogen carbonate, potassium hydrogen carbonate, lithium carbonate, lithium hydrogen carbonate, sodium hydroxide, potassium hydroxide, lithium hydroxide, calcium hydroxide, magnesium hydroxide, trisodium phosphate, disodium hydrogen phosphate, tripotassium hydrogen phosphate, dipotassium hydrogen phosphate, triethylamine, diethylamine, 1,8-diazabicyclo[5.4.0]undeca-7-ene, 1,5-diazabicyclo[4.3.0]-5-nonene, 1,4-diazabicyclo[2.2.2]octane, pyridine, 2-amino-2-methyl-1-propanol, or the like. These weak bases or bases may be used alone or in combination of two or more types thereof.

The weak base or base is preferably sodium carbonate, potassium carbonate, sodium hydrogen carbonate, potassium hydrogen carbonate, disodium hydrogen phosphate, or dipotassium hydrogen phosphate, which is a weak base, more preferably sodium hydrogen carbonate, potassium carbonate, or potassium hydrogen carbonate, depending on the type of the solution and the like.

### [2. Step (2)]

The method for producing a heteropolyacid salt having a modified lacunary site or a mixture thereof according to the present embodiment includes, as a step (2), (2) a step of reacting a mono-lacunary Keggin-type or Dawson-type heteropolyacid salt with a P, Si, Ge, or Sn compound having one or more hydro groups or organic groups and two or more leaving groups to obtain a heteropolyacid salt having a modified lacunary site represented by the following general formula (I) or a mixture thereof.

(A^{p+})_{q}[C^{(pxq)-}] (I)

[wherein
A^{p+} each independently denotes H⁺, a metal ion, or an ammonium ion,
C^{(pxq)-} denotes a mono-lacunary Keggin-type or Dawson-type heteropolyacid anion, the heteropolyacid anion having a modified lacunary site, and
p denotes an integer in the range of 1 to 5, and q denotes a real number]

The step (2) may include, as a step (2a), a step of reacting the mono-lacunary Keggin-type or Dawson-type heteropolyacid salt with the P, Si, Ge, or Sn compound having one or more hydro groups or organic groups and two or more leaving groups in a solution with a pH of 5 or less.

### <P, Si, Ge, or Sn Compound Having One or More Hydro Groups or Organic Groups and Two or More Leaving Groups>

The P, Si, Ge, or Sn compound having one or more hydro groups or organic groups and two or more leaving groups is not particularly limited and may be a known and commonly used compound. The P, Si, Ge, or Sn compound having one or more hydro groups or organic groups and two or more leaving groups is, for example, but not limited to, a P, Si, Ge, or Sn compound having a hydro group or an organic group and two or more leaving groups represented by any one of the general formulae (IV-1) to (IV-4).

(R^{1A})X'Y₃ (IV-1)

(R^{1B1})(R^{1B2})X'Y₂ (IV-2)

(R^{1C})P(=O)Y₂ (IV-3)

(R^{1D})X''Y₃ (IV-4)

[In the formulae (IV-1) to (IV-4),
X' denotes a heteroatom of Si or Ge,
X'' denotes a heteroatom of Si, Ge, or Sn,
R^{1A} to R^{1D} each independently denote a hydro group, an optionally substituted C₁₋₁₈ hydrocarbyl group, an optionally substituted 3- to 18-membered non-aromatic heterocyclic group, or an optionally substituted 5- to 18-membered aromatic heterocyclic group,
a divalent carbon atom at any position of R^{1A} to R^{1D} except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{1A} to R^{1D} may be substituted with, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group, and
Y denotes a leaving group]

The leaving group may be a known and commonly used leaving group. The leaving group is, for example, but not limited to, a halogen atom, a hydroxy group, an alkoxy group (preferably a C₁₋₄ alkoxy group, more preferably a C₁₋₂ alkoxy group), an alkylcarbonyloxy group (preferably a C₁₋₄ alkylcarbonyloxy group), or a hydrocarbylsulfonyloxy group (preferably a C₁₋₆ alkyl or a C₇₋₁₀ aralkylsulfonyloxy group; for example, a methanesulfonyloxy group, a p-toluenesulfonyloxy group, or the like), or the like.

### (Organic Group Having One or More Substituents, such as Hydroxy Group, Halogen Atom, or Epoxy Group)

From the perspective of preparing a functional building block, R^{1A} to R^{1D} in the general formulae (IV-1) to (IV-4) are
preferably optionally substituted C₁₋₁₈ hydrocarbyl groups; any divalent carbon atom at a non-terminal position of R^{1A} to R^{1D} may be substituted with -O-, -C(=O)-, -C(=O)O-, - OCO-, -CONH-, -NHCO-, -NH(C=O)O-, -S-, or -SO₂- (provided that adjacent divalent carbon atoms are not substituted at the same time), and one or more hydrogen atoms in R^{1A} to R^{1D} is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (o) an epoxy group, (p) a glycidyl group, or (r) an amide group,
more preferably C₁₋₁₈ alkyl groups, C₃₋₁₈ alicyclic groups, or C₇₋₁₈ aralkyl groups, each optionally having a substituent; any divalent carbon atom at a non-terminal position of R^{1A} to R^{1D} may be substituted with -O-, -C(=O)-, -C(=O)O-, -OCO-, -CONH-, -NHCO-, - NH(C=O)O-, -S-, or -SO₂- (provided that adjacent divalent carbon atoms are not substituted at the same time), and one or more hydrogen atoms in R^{1A} to R^{1D} is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (o) an epoxy group, (p) a glycidyl group, or (r) an amide group.

Specific examples of R^{1A} to R^{1D} are not particularly limited and include a 1-glycidyloxypropan-3-yl group, a 1-glycidyloxy-n-octan-8-yl group, a 1-aminopropan-3-yl group, a 1-(2-aminoethylamino)-propan-3-yl group, a 3,3,3-trifluoropropan-1-yl group, a chloromethyl group, a 1-chloropropan-3-yl group, a 1-bromopropan-3-yl group, a 1-iodopropan-3-yl group, and the like.

### <Organic Group Having One or More Ethylenically Unsaturated Double Bonds>

Furthermore, from the perspective of preparing a building block having actinic radiation reactivity, the organic group preferably includes at least one ethylenically unsaturated double bond.

That is, R^{1A} to R^{1D} in the general formulae (IV-1) to (IV-4) are
preferably optionally substituted C₁₋₁₈ hydrocarbyl groups; any divalent carbon atom at a non-terminal position of R^{1A} to R^{1D} may be substituted with -O-, -C(=O)-, -C(=O)O-, - OCO-, -CONH-, -NHCO-, -NH(C=O)O-, -S-, or -SO₂- (provided that adjacent divalent carbon atoms are not substituted at the same time), and one or more hydrogen atoms in R^{1A} to R^{1D} is substituted with (j) a vinyl group, (k) an allyl group, (l) a (meth)acryloyl group, (m) a (meth)acryloyloxy group, (o) a (meth)acrylamide group, or (p) a styryl group,
more preferably C₁₋₁₈ alkyl groups, C₃₋₁₈ alicyclic groups, or C₇₋₁₈ aralkyl groups, each optionally having a substituent; any divalent carbon atom at a non-terminal position of R^{1A} to R^{1D} may be substituted with -O-, -C(=O)-, -C(=O)O-, -OCO-, -CONH-, -NHCO-, - NH(C=O)O-, -S-, or -SO₂- (provided that adjacent divalent carbon atoms are not substituted at the same time), and one or more hydrogen atoms in R^{1A} to R^{1D} is substituted with (j) a vinyl group, (k) an allyl group, (l) a (meth)acryloyl group, (m) a (meth)acryloyloxy group, (o) a (meth)acrylamide group, or (p) a styryl group.

Specific examples of the organic groups R^{1A} to R^{1D} having the ethylenically unsaturated double bond are not particularly limited and include a vinyl group, an allyl group, a 5-hexen-1-yl group, a 6-hepten-1-yl group, a 7-octen-1-yl group, a 1-acryloyloxypropan-3-yl group, a 1-methacryloyloxypropan-3-yl group, a 1-vinylbenzene-4-yl group, and the like.

### <Organic Group Having One or More Hydroxy Groups or Carboxy Groups Having Protective Group>

Furthermore, from the perspective of providing a building block whose functionality, such as solubility, is changed by deprotection due to the action of an acid or the like, the organic group preferably has one or more hydroxy groups or carboxy groups, each having a protective group.

That is, R^{1A} to R^{1D} in the general formulae (IV-1) to (IV-4) are
preferably optionally substituted C₁₋₁₈ hydrocarbyl groups; any divalent carbon atom at a non-terminal position of R^{1A} to R^{1D} may be substituted with -O-, -C(=O)-, -C(=O)O-, - OCO-, -CONH-, -NHCO-, -NH(C=O)O-, -S-, or -SO₂- (provided that adjacent divalent carbon atoms are not substituted at the same time), and one or more hydrogen atoms in R^{1A} to R^{1D} are substituted with (s) a hydroxy group or a carboxy group each having a protective group,
more preferably C₁₋₁₈ alkyl groups, C₃₋₁₈ alicyclic groups, or C₇₋₁₈ aralkyl groups, each optionally having a substituent; any divalent carbon atom at a non-terminal position of R^{1A} to R^{1D} may be substituted with -O-, -C(=O)-, -C(=O)O-, -OCO-, -CONH-, -NHCO-, - NH(C=O)O-, -S-, or -SO₂- (provided that adjacent divalent carbon atoms are not substituted at the same time), and one or more hydrogen atoms in R^{1A} to R^{1D} are substituted with (s) a hydroxy group or a carboxy group each having a protective group.

The protective group for the hydroxy group or the carboxy group may be, but not limited to, an ether protective group, a silyl ether protective group, an acetal protective group, an acyl protective group, an oxycarbonyl (alkyl) protective group, a dihydrocarbylaminocarbonyl protective group, or the like.

The protective group for the hydroxy group or the carboxy group is preferably an ether protective group, such as a t-butyl group or a benzyl group; a silyl ether protective group, such as a t-butyldimethylsilyl group, a triisopropylsilyl group, a trimethylsilyl group, a triethylsilyl group, or a t-butyldiphenylsilyl group; an acetal protective group, such as a methoxymethyl group, an ethoxyethyl group, a 2-tetrahydropyranyl group, or a methoxyethoxymethyl group; an acyl protective group, such as an acetyl group, a pivaloyl group, or a benzoyl group; an oxycarbonyl (alkyl) protective group, such as a t-butoxycarbonyl group; a dihydrocarbylaminocarbonyl protective group, such as a dimethylaminocarbonyl group, a diethylaminocarbonyl group, a diisopropylaminocarbonyl group, or an N-phenyl-N-methyl-aminocarbonyl group; or an acid-dissociable group in which carbon bonded to a hydroxy group or a carboxy group is tertiary carbon,
more preferably an acetal protective group, such as a methoxymethyl group, an ethoxyethyl group, a 2-tetrahydropyranyl group, or a methoxyethoxymethyl group; an oxycarbonyl (alkyl) protective group, such as a t-butoxycarbonyl group; a dihydrocarbylaminocarbonyl protective group, such as a dimethylaminocarbonyl group, a diethylaminocarbonyl group, a diisopropylaminocarbonyl group, or an N-phenyl-N-methyl-aminocarbonyl group; or an acid-dissociable group in which carbon bonded to a hydroxy group or a carboxy group is tertiary carbon and that has a cyclopentane skeleton, a cyclohexane skeleton, a cycloheptane skeleton, a norbornane skeleton, or an adamantane skeleton.

The protective group is, for example, but not limited to, a methoxymethyl group, an ethoxyethyl group, a methoxyethoxymethyl group, a t-butoxycarbonyl group, a dimethylaminocarbonyl group, a diethylaminocarbonyl group, a diisopropylaminocarbonyl group, an N-phenyl-N-methyl-aminocarbonyl group, a 1-methylcyclopentyl group, a 1-ethylcyclopentyl group, a 1-phenylcyclopentyl group, a 1-tolylcyclopentyl group, a 1-methylcyclohexyl group, a 1-ethylcyclohexyl group, a 1-phenylcyclohexyl group, a 1-tolylcyclohexyl group, a 1-(naphthalen-2-yl)cyclohexyl group, a 1-(1-methoxy-2-methylpropan-2-yl)cyclopentyl group, a 1-(1-ethoxy-2-methylpropan-2-yl)cyclopentyl group, a 2-methyladamantan-2-yl group, a 2-ethyladamantan-2-yl group, a 2-phenyladamantan-2-yl group, or the like.

### <Heteropolyacid Salt Having Modified Lacunary site or Mixture Thereof (I)>

The mono-lacunary Keggin-type or Dawson-type heteropolyacid salt is reacted with the P, Si, Ge, or Sn compound having one or more hydro groups or organic groups and two or more leaving groups to yield a heteropolyacid salt having a modified lacunary site or a mixture thereof.

The state in which the lacunary site is modified with the P, Si, Ge, or Sn compound having one or more hydro groups or organic groups and two or more leaving groups represented by the general formulae (IV-1) to (IV-4) can be represented by the following general formulae (V-1) to (V-4). 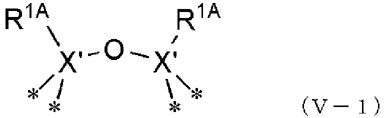 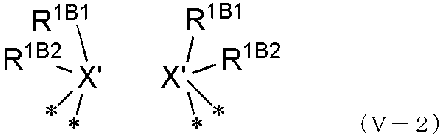 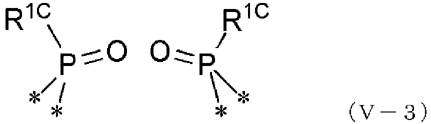 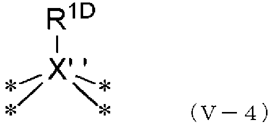 [In the formulae (V-1) to (V-4),
X' denotes Si or Ge,
X" denotes a heteroatom of Si, Ge, or Sn,
R^{1A} to R^{1D} each independently denote a hydro group, an optionally substituted C₁₋₁₈ hydrocarbyl group, an optionally substituted 3- to 18-membered non-aromatic heterocyclic group, or an optionally substituted 5- to 18-membered aromatic heterocyclic group,
a divalent carbon atom at any position of R^{1A} to R^{1D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{1A} to R^{1D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group, and
* denotes the binding portion to a terminal oxygen atom of the lacunary site.]

In the present description, the heteropolyacid anion having the lacunary site modified with the P, Si, Ge, or Sn compound having one or more hydro groups or organic groups and two or more leaving groups represented by the general formulae (V-1) to (V-4) may be represented by the following general formulae (VI-1) to (VI-8) for the sake of convenience.

[XM₁₁O₃₉(R^{1A}X')₂O]^{c31-} (VI-1)

[XM₁₁O₃₉(R^{1B1}R^{1B2}X')₂]^{c31-} (VI-2)

[XM₁₁O₃₉(R^{1C}P=O)₂]^{c31-} (VI-3)

[XM₁₁O₃₉(R^{1D}X")]^{c31-} (VI-4)

[X₂M₁₇O₆₁(R^{1A}X')₂O]^{c32-} (VI-5)

[X₂M₁₇O₆₁(R^{1B1}R^{1B2}X')₂]^{c32-} (VI-6)

[X₂M₁₇O₆₁(R^{1C}P=O)₂]^{c32-} (VI-7)

[X₂M₁₇O₆₁(R^{1D}X")]^{c32-} (VI-8)

[wherein
X denotes a heteroatom of P, Si, B, S, or Ge,
M denotes a polyatom of Mo, W, V, Nb, or Ta, and
X' denotes a heteroatom of Si or Ge,
X" denotes a heteroatom of Si, Ge, or Sn,
R^{1A} to R^{1D} each independently denote a hydro group, an optionally substituted C₁₋₁₈ hydrocarbyl group, an optionally substituted 3- to 18-membered non-aromatic heterocyclic group, or an optionally substituted 5- to 18-membered aromatic heterocyclic group,
a divalent carbon atom at any position of R^{1A} to R^{1D} except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{1A} to R^{1D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group, and
c31 and c32 are natural numbers]

In the heteropolyacid anion having the modified lacunary site, since four terminal oxygen atoms of the lacunary site are modified, in general, the value of c31 is c21 - 4, and the value of c32 is c22 - 4.

On the other hand, for example, when the organic group contains an amino group and the amino group is protonated to be an ammonium cation, or when the organic group contains a carboxy group and the carboxy group is converted into a carboxy anion, the values of c31 and c32 are different from the above values.

### [2-1. Step (2a)]

The step (2) may include, as a step (2a), a step of reacting the mono-lacunary Keggin-type or Dawson-type heteropolyacid salt with the P, Si, Ge, or Sn compound having one or more hydro groups or organic groups and two or more leaving groups in a solution with a pH of 5 or less.

The step (2) including the step (2a) can further enhance the reaction rate and efficiency of the reaction for modifying the lacunary site of the mono-lacunary Keggin-type or Dawson-type heteropolyacid salt with the P, Si, Ge, or Sn compound having one or more hydro groups or organic groups and two or more leaving groups.

The solution in the step (2a) is, for example, but not limited to, water and/or one or more organic solvents similar to those exemplified in the step (1a).

The pH of the solution in the step (2a) is preferably 5 or less, more preferably 4.8 or less, even more preferably -1 to 4.5, still more preferably -1 to 4.0, particularly preferably 0 to 4.

The method for adjusting the pH of the solution to 5 or less in the step (2a) is, for example, but is not limited to, a method of adding an acidic solution, or the like.

The type of acid contained in the acidic solution is, for example, an inorganic acid, such as hydrochloric acid, sulfuric acid, nitric acid, sulfamic acid, or phosphoric acid; an organic acid having a carboxy group, such as tartaric acid, oxalic acid, formic acid, acetic acid, propionic acid, malic acid, lactic acid, citric acid, succinic acid, maleic acid, fumaric acid, glycolic acid, trifluoroacetic acid, or trichloroacetic acid; an organic acid having a sulfo group, such as methanesulfonic acid, ethanesulfonic acid, sulfosuccinic acid, m-toluenesulfonic acid, or p-toluenesulfonic acid; or the like. These acids may be used alone or in combination of two or more types thereof.

The acid is preferably hydrochloric acid, sulfuric acid, nitric acid, phosphoric acid, or acetic acid, more preferably hydrochloric acid, sulfuric acid, or acetic acid, depending on the type of the solution and the like.

### [3. Step (3)]

The method for producing a heteropolyacid salt having a modified lacunary site or a mixture thereof according to the present embodiment has, as a step (3), (3) a step of salt-exchanging the heteropolyacid salt having the modified lacunary site represented by the general formula (I) or a mixture thereof.

The step (3) may include, as a step (3a), a step of salt-exchanging the heteropolyacid salt having the modified lacunary site represented by the general formula (I) or a mixture thereof with an alkali metal salt, an alkaline-earth metal salt, an aluminum salt, or an onium salt.

The step (3) may also include, after the step (3a), as a step (3b), a step of obtaining a heteropolyacid salt having a modified lacunary site represented by the following general formula (VII) or a mixture thereof.

(A^{m+})ₐ(Bⁿ⁺)_{b}(C^{(am+bn)-}) (VII)

[wherein
when b is 0,
A^{m+} each independently denotes H⁺, a metal ion, or an ammonium ion, at least one of which is an alkali metal ion, an alkaline-earth metal ion, or Al³⁺, different from A^{p+},
when b is a real number greater than 0,
A^{m+} each independently denotes H⁺, a metal ion, or an ammonium ion,
Bⁿ⁺ each independently denotes an organic sulfonium cation, an organic sulfonium dication, an organic iodonium cation, an organic ammonium cation, an organic ammonium dication, an organic phosphonium cation, or an organic phosphonium dication,
C^{(am+bn)-} denotes a mono-lacunary Keggin-type or Dawson-type heteropolyacid anion, the heteropolyacid anion having a modified lacunary site, and
m denotes an integer in the range of 1 to 5, n denotes an integer of 1 or 2, and a is a real number]

### [3-1. Step (3a)]

The step (3) may include, as a step (3a), a step of salt-exchanging the heteropolyacid salt having the modified lacunary site represented by the general formula (I) or a mixture thereof with an alkali metal salt, an alkaline-earth metal salt, an aluminum salt, or an onium salt.

### <Alkali Metal Salt, Alkaline-earth Metal Salt, or Aluminum Salt>

The metal cation of the alkali metal salt, alkaline-earth metal salt, or aluminum salt is, for example, but not limited to, an alkali metal ion, such as Li⁺, Na⁺, K⁺, Rb⁺, or Cs⁺; an alkaline-earth metal ion, such as Be²⁺, Mg²⁺, Ca²⁺, Sr²⁺, or Ba²⁺; Al³⁺, or the like.

The counter anion of the alkali metal salt, alkaline-earth metal salt, or aluminum salt is, for example, but not limited to, Cl⁻, Br⁻, I⁻, OH⁻, CF₃SO₃⁻, C₂F₅SO₃⁻, C₃F₇SO₃⁻, C₄F₉SO₃⁻, p-CH₃(C₆H₄)SO₃⁻, ClSO₃⁻, ClO₄⁻, (C₆F₅)₄B⁻, BF₄⁻, PF₆⁻, SbF₆⁻, AlCl₄⁻, BiF₆⁻, AsF₆⁻, or the like.

Specific examples of alkali metal salt, alkaline-earth metal salt, or aluminum salt are, for example, sodium chloride, potassium chloride, rubidium chloride, cesium chloride, magnesium chloride, calcium chloride, strontium chloride, barium chloride, aluminum chloride, sodium bromide, potassium bromide, rubidium bromide, cesium bromide, magnesium bromide, calcium bromide, strontium bromide, barium bromide, aluminum bromide, sodium iodide, potassium iodide, rubidium iodide, cesium iodide, magnesium iodide, calcium iodide, strontium iodide, barium iodide, aluminum iodide, or the like.

### <Onium Salt>

The onium salt is, for example, but not limited to, an organic sulfonium salt, an organic iodonium salt, an organic ammonium salt, or an organic phosphonium salt, or the like. The cation moiety and the anion moiety of the onium salt will be separately described below.

### <<Cation Moiety of Onium Salt>>

### (Cation Moiety of Organic Sulfonium Salt)

The cation moiety of the organic sulfonium salt can be an organic sulfonium cation or an organic sulfonium dication.

The organic sulfonium cation can be an organic sulfonium cation represented by the following general formula (VIII).

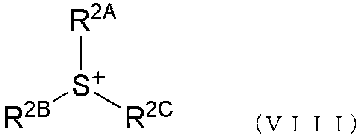

In the formula (VIII), R^{2A}, R^{2B}, and R^{2C} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{2A}, R^{2B}, and R^{2C} except the terminal positions may be substituted with -O-, -C(=O)-, -C(=O)O-, -OCO-, -CONH-, -NHCO-, - NH(C=O)O-, -S-, or -SO₂- (provided that adjacent divalent carbon atoms are not substituted at the same time),
a hydrogen atom in R^{2A}, R^{2B}, and R^{2C} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
any two of R^{2A}, R^{2B}, and R^{2C} may be directly bonded to each other by a single bond or may be bonded to each other via a divalent linking group -O-, -S-, -C(=O)-, -S(=O)-, - S(=O)₂-, -C(=O)O-, or a C₁₋₃ alkylene group to form a ring together with the sulfur atom in the formula (VIII).

In the organic sulfonium cation represented by the general formula (VIII), from the perspective of providing a functional building block,
R^{2A}, R^{2B}, and R^{2C} each independently preferably denote
an optionally substituted C₁₋₁₈ hydrocarbyl group; a divalent carbon atom at any position of R^{2A}, R^{2B}, and R^{2C} except the terminal positions may be substituted with -O-, - C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{2A}, R^{2B}, and R^{2C} is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group,
more preferably a C₁₋₁₈ alkyl group, a C₃₋₁₈ alicyclic group, a C₆₋₁₈ aryl group, or a C₇₋₁₈ aralkyl group, each optionally having a substituent; a divalent carbon atom at any position of R^{2A}, R^{2B}, and R^{2C} except the terminal positions may be substituted with -O-, - C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{2A}, R^{2B}, and R^{2C} is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.

The organic sulfonium dication can be an organic sulfonium dication represented by the following general formula (IX).

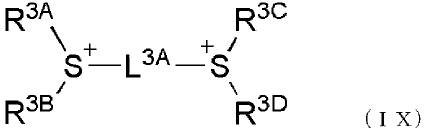

In the formula (IX), R^{3A} to R^{3D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{3A} to R^{3D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
L^{3A} denotes an optionally substituted C₁₋₁₈ hydrocarbylene group,
a divalent carbon atom at any position of L^{3A} may be substituted with -O-, -C(=O)-, -C(=O)O-, -OCO-, -CONH-, -NHCO-, -S-, or -SO₂- (provided that adjacent divalent carbon atoms are not substituted at the same time), and
a hydrogen atom in R^{3A} to R^{3D} and L^{3A} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
any two of R^{3A} to R^{3D} and L^{3A} may be directly bonded to each other by a single bond or may be bonded to each other via a divalent linking group -O-, -S-, -C(=O)-, -S(=O)-, - S(=O)₂-, -C(=O)O-, or a C₁₋₃ alkylene group to form a ring together with the sulfur atom in the formula (IX).

### (Cation Moiety of Organic Iodonium Salt)

The cation moiety of the organic iodonium salt can be an organic iodonium cation represented by the following general formula (X).

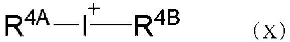

In the general formula (X), R^{4A} and R^{4B} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{4A} and R^{4B} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{4A} and R^{4B} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbyloxycarbonyloxy group, in which at least part of hydrogen atoms may be substituted with (a) to (s), and a divalent carbon atom at any position of these substituents except the terminal positions may be substituted with -O-, -C(=O)-, -C(=O)O-, -OCO-, - CONH-, -NHCO-, -NH(C=O)O-, -S-, or -SO₂- (provided that adjacent divalent carbon atoms are not substituted at the same time), and
R^{4A} and R^{4B} may be directly bonded to each other by a single bond or may be bonded to each other via a divalent linking group -O-, -S-, -C(=O)-, -S(=O)-, -S(=O)₂-, -C(=O)O-, or a C₁₋₃ alkylene group to form a ring together with the iodine atom in the formula (X).

In the organic iodonium cation represented by the general formula (X), from the perspective of providing a functional building block,
R^{4A} and R^{4B} each independently preferably denote
an optionally substituted C₁₋₁₈ hydrocarbyl group; a divalent carbon atom at any position of R^{4A}, and R^{4B} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{4A}, and R^{4B} is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group,
more preferably a C₁₋₁₈ alkyl group, a C₃₋₁₈ alicyclic group, a C₆₋₁₈ aryl group, or a C₇₋₁₈ aralkyl group, each optionally having a substituent; a divalent carbon atom at any position of R^{4A}, and R^{4B} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{4A}, and R^{4B} is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.

### (Cation Moiety of Organic Ammonium Salt)

The cation moiety of the organic ammonium salt can be an organic ammonium cation or an organic ammonium dication. The organic ammonium cation is, for example, but not limited to, an organic primary ammonium cation (NH₃(R^{5A})⁺), an organic secondary ammonium cation (NH₂(R^{5A})(R^{5B})⁺), an organic tertiary ammonium cation (NH(R^{5A})(R^{5B})(R^{5C})⁺), or an organic quaternary ammonium cation (N(R^{5A})(R^{5B})(R^{5C})(R^{5D})⁺). R^{5A} to R^{5D} denote the same as defined below.

The organic ammonium cation is preferably an organic tertiary ammonium cation or an organic quaternary ammonium cation, more preferably an organic quaternary ammonium cation.

The organic quaternary ammonium cation can be an organic quaternary ammonium cation represented by the following general formula (XI).

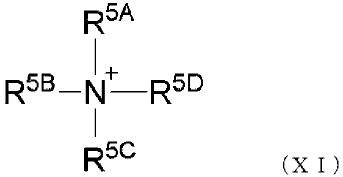

In the general formula (XI), R^{5A} to R^{5D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{5A} to R^{5D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{5A} to R^{5D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbyloxycarbonyloxy group, in which at least part of hydrogen atoms may be substituted with (a) to (s), and a divalent carbon atom at any position of these substituents except the terminal positions may be substituted with -O-, -C(=O)-, -C(=O)O-, -OCO-, - CONH-, -NHCO-, -NH(C=O)O-, -S-, or -SO₂- (provided that adjacent divalent carbon atoms are not substituted at the same time), and
any two of R^{5A} to R^{5D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the nitrogen atom in the formula (XI).

In the organic quaternary ammonium cation represented by the general formula (XI), from the perspective of providing a functional building block,
R^{5A} to R^{5D} each independently denote
a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{5A} to R^{5D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
at least one hydrogen atom in R^{5A} to R^{5D} is preferably substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group,
R^{5A} to R^{5D} each independently denote a C₁₋₁₈ alkyl group, a C₃₋₁₈ alicyclic group, a C₆₋₁₈ aryl group, or a C₇₋₁₈ aralkyl group, each optionally having a substituent,
a divalent carbon atom at any position of R^{5A} to R^{5D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
at least one hydrogen atom in R^{5A} to R^{5D} is more preferably substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.

The organic ammonium dication is not particularly limited and may be a known and commonly used organic ammonium dication. The organic ammonium dication is, for example, an organic ammonium dication having two organic primary to quaternary ammonium cations. The organic quaternary ammonium dication having two organic quaternary ammonium cations can be an organic quaternary ammonium dication represented by the following general formula (XII).

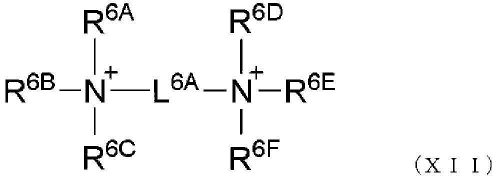

In the formula (XII), R^{6A} to R^{6F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{6A} to R^{6F} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
L^{6A} denote an optionally substituted C₁₋₁₈ hydrocarbylene group,
a divalent carbon atom at any position of L^{6A} may be substituted with -O-, -C(=O)-, -C(=O)O-, -OCO-, -CONH-, -NHCO-, -S-, or -SO₂- (provided that adjacent divalent carbon atoms are not substituted at the same time), and
a hydrogen atom in R^{6A} to R^{6F} and L^{6A} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbyloxycarbonyloxy group, in which at least part of hydrogen atoms may be substituted with (a) to (s), and a divalent carbon atom at any position of these substituents except the terminal positions may be substituted with -O-, -C(=O)-, -C(=O)O-, -OCO-, - CONH-, -NHCO-, -NH(C=O)O-, -S-, or -SO₂- (provided that adjacent divalent carbon atoms are not substituted at the same time), and
any two of R^{6A} to R^{6F} and L^{6A} may be directly bonded to each other by a single bond or may be bonded to each other via a divalent linking group -O-, -S-, -C(=O)-, -S(=O)-, - S(=O)₂-, -C(=O)O-, or a C₁₋₃ alkylene group to form a ring together with the nitrogen atom in the formula (XII).

### (Cation Moiety of Organic Phosphonium Salt)

The cation moiety of the organic phosphonium salt can be an organic phosphonium cation or an organic phosphonium dication.

The organic phosphonium cation can be an organic phosphonium cation represented by the following general formula (XIII).

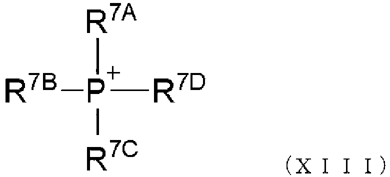

In the formula (XIII), R^{7A} to R^{7D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{7A} to R^{7D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
a hydrogen atom in R^{7A} to R^{7D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbyloxycarbonyloxy group,, in which at least part of hydrogen atoms may be substituted with (a) to (s), and a divalent carbon atom at any position of these substituents except the terminal positions may be substituted with -O-, -C(=O)-, -C(=O)O-, -OCO-, - CONH-, -NHCO-, -NH(C=O)O-, -S-, or -SO₂- (provided that adjacent divalent carbon atoms are not substituted at the same time), and
any two of R^{7A} to R^{7D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the phosphoric atom in the formula (XIII).

In the organic quaternary phosphonium cation represented by the general formula (XIII), from the perspective of providing a functional building block,
R^{7A} to R^{7D} each independently denote
a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{7A} to R^{7D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
at least one hydrogen atom in R^{7A} to R^{7D} is preferably substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group,
R^{7A} to R^{7D} each independently denote a C₁₋₁₈ alkyl group, a C₃₋₁₈ alicyclic group, a C₆₋₁₈ aryl group, or a C₇₋₁₈ aralkyl group, each optionally having a substituent,
a divalent carbon atom at any position of R^{7A} to R^{7D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
at least one hydrogen atom in R^{7A} to R^{7D} is more preferably substituted with (a) a halogen atom, (b) haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.

The organic phosphonium dication can be an organic phosphonium dication represented by the following general formula (XIV).

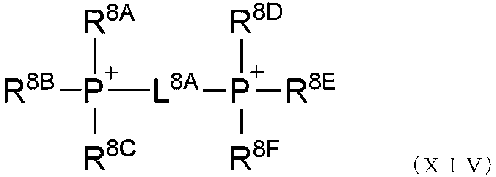

In the formula (XIV), R^{8A} to R^{8F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{8A} to R^{8F} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
L^{8A} denote an optionally substituted C₁₋₁₈ hydrocarbylene group,
a divalent carbon atom at any position of L^{8A} may be substituted with -O-, -C(=O)-, -C(=O)O-, -OCO-, -CONH-, -NHCO-, -S-, or -SO₂- (provided that adjacent divalent carbon atoms are not substituted at the same time), and
a hydrogen atom in R^{8A} to R^{8F} and L^{8A} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbyloxycarbonyloxy group, in which at least part of hydrogen atoms may be substituted with (a) to (s), and a divalent carbon atom at any position of these substituents except the terminal positions may be substituted with -O-, -C(=O)-, -C(=O)O-, -OCO-, - CONH-, -NHCO-, -NH(C=O)O-, -S-, or -SO₂- (provided that adjacent divalent carbon atoms are not substituted at the same time), and
any two of R^{8A} to R^{8F} and L^{8A} may be directly bonded to each other by a single bond or may be bonded to each other via a divalent linking group -O-, -S-, -C(=O)-, -S(=O)-, - S(=O)₂-, -C(=O)O-, or a C₁₋₃ alkylene group to form a ring together with the phosphoric atom in the formula (XIV).

### <<Anion Moiety of Onium Salt>>

The anion moiety of the onium salt is, for example, but not limited to, a Cl⁻, Br⁻, I⁻, OH⁻, CF₃SO₃⁻, C₂F₅SO₃⁻, C₃F₇SO₃⁻, C₄F₉SO₃⁻, p-CH₃(C₆H₄)SO₃⁻, ClSO₃⁻, ClO₄⁻, (C₆F₅)₄B⁻, BF₄⁻, PF₆⁻, SbF₆⁻, AlCl₄⁻, BiF₆⁻, AsF₆⁻, or the like.

From the perspective of promoting the salt exchange reaction, the anion moiety of the onium salt is preferably Cl⁻, Br⁻, I⁻, OH⁻, CF₃SO₃⁻, C₄F₉SO₃⁻, BF₄⁻, PF₆⁻, SbF₆⁻, more preferably Cl⁻, Br⁻, CF₃SO₃⁻, BF₄⁻, PF₆⁻, or SbF₆⁻.

### <<Examples of Onium Salt>>

Specific examples of the onium salt include organic sulfonium salts, such as methioninemethylsulfonium chloride, (2-bromoethyl)diphenylsulfonium trifluoromethanesulfonate, benzyl(4-hydroxyphenyl)methylsulfonium hexafluoroantimonate, (2-carboxyethyl)dimethylsulfonium bromide, dimesityl(trifluoromethyl)sulfonium trifluoromethanesulfonate, diphenyl(4-(phenylthio)phenyl)sulfonium hexafluoroantimonate, 5-vinyl-5H-thianthren-5-ium tetrafluoroborate, triphenylsulfonium tetrafluoroborate, tri-p-tolylsulfonium trifluoromethanesulfonate, and (thiodi-4,1-phenylene)bis(diphenylsulfonium)bis(hexafluorophosphate); organic iodonium salts, such as bis(4-t-butylphenyl)iodonium hexafluorophosphate, bis(4-bromophenyl)iodonium trifluoromethanesulfonate, bis(2,4,6-trimethylphenyl)iodonium trifluoromethanesulfonate, (3-bromophenyl)(mesityl)iodonium trifluoromethanesulfonate, diphenyliodonium chloride, (2-methylphenyl)(2,4,6-trimethylphenyl)iodonium trifluoromethanesulfonate, (4-nitrophenyl)(phenyl)iodonium trifluoromethanesulfonate, phenyl(3-(trifluoromethyl)phenyl)iodonium trifluoromethanesulfonate, (3-(trifluoromethyl)phenyl)(2,4,6-trimethylphenyl)iodonium trifluoromethanesulfonate, (4-(trifluoromethyl)phenyl)(2,4,6-trimethylphenyl)iodonium trifluoromethanesulfonate, bis(4-fluorophenyl)iodonium trifluoromethanesulfonate, ((4-trifluoromethyl)phenyl)(2,4,6-trimethoxyphenyl)iodonium p-toluenesulfonate, 4-biphenylyl(2,4,6-trimethoxyphenyl)iodonium triflumethanesulfonate, and (3,5-dichlorophenyl)(2,4,6-trimethoxyphenyl)iodonium p-toluenesulfonate; organic quaternary ammonium salts, such as benzyltrimethylammonium chloride, tetrabutylammonium chloride, dodecyltrimethylammonium chloride, acetylcholine chloride, benzoylcholine chloride, benzyldimethylphenylammonium chloride, bis(2-hydroxyethyl)dimethylammonium chloride, bethanechol chloride, (3-acrylamidopropyl)trimethylammonium chloride, N-(2-acryloyloxyethyl)-N-benzyl-N,N-dimethylammonium chloride, diallyldimethylammonium chloride, glycidyltrimethylammonium chloride, methacryloylcholine chloride, hexyltrimethylammonium bromide, 1-methyl-1-propylpiperidinium bromide, 3-(trifluoromethyl)phenyltrimethylammonium bromide, trimethylvinylammonium bromide, acetylthiocholine iodide, (3-bromopropyl)trimethylammonium bromide, (2-hydroxyethyl)triethylammonium iodide, hexamethonium chloride, and decamethonium bromide; organic phosphonium salts, such as (methoxymethyl)triphenylphosphonium chloride, tetrabutylphosphonium bromide, amyltriphenylphosphonium bromide, allyltriphenylphosphonium bromide, acetonyltriphenylphosphonium chloride, benzyltriphenylphosphonium chloride, butyltriphenylphosphonium bromide, (bromomethyl)triphenylphosphonium bromide, 3-bromopropyltriphenylphosphonium bromide, (4-bromobenzyl)triphenylphosphonium bromide, (chloromethyl)triphenylphosphonium chloride, 4-(carboxybutyl)triphenylphosphonium bromide, (cyanomethyl)triphenylphosphonium chloride, (2-chlorobenzyl)triphenylphosphonium chloride, 2-(1,3-dioxan-2-yl)ethyltriphenylphosphonium bromide, (2,4-dichlorobenzyl)triphenylphosphonium chloride, ethyltriphenylphosphonium iodide, triphenylpropargylphosphonium bromide, tetraphenylphosphonium bromide, cyclopropyltriphenylphosphonium bromide, and trans-2-butene-1,4-bis(triphenylphosphonium chloride); and the like.

### <Salt Exchange Reaction>

The reaction conditions for the salt exchange between the heteropolyacid salt having a modified lacunary site represented by the general formula (I) or a mixture thereof and the alkali metal salt, alkaline-earth metal salt, aluminum salt, or onium salt are not particularly limited, and the reaction can be carried out under known and commonly used reaction conditions, and the type of solvent, the reaction temperature, the reaction time, the pressure, and the like can be appropriately selected.

The type of solvent is, for example, but not limited to, water, methanol, ethanol, 1,4-dioxane, acetonitrile, 1-propanol, 2-propanol, ethyl acetate, acetonitrile, acetone, dichloromethane, chloroform, dimethylformamide, diethyl ether, or the like. These solvents may be used alone or in combination of two or more types thereof.

The reaction temperature is, for example, but not limited to, room temperature or under heating.

The reaction time is not particularly limited and preferably ranges from 1 minute to 24 hours, more preferably 1 minute to 5 hours.

The pressure may be, but is not limited to, atmospheric pressure or under pressure.

### [3-2. Step (3b)]

The step (3) may include, after the step (3a), as a step (3b), a step of obtaining a heteropolyacid salt having a modified lacunary site represented by the general formula (VII) or a mixture thereof.

(A^{m+})ₐ(Bⁿ⁺)_{b}(C^{(am+bn)-}) (VII)

[wherein
when b is 0,
A^{m+} each independently denotes H⁺, a metal ion, or an ammonium ion, at least one of which is an alkali metal ion, an alkaline-earth metal ion, or Al³⁺, different from A^{p+},
when b is a real number greater than 0,
A^{m+} each independently denotes H⁺, a metal ion, or an ammonium ion,
Bⁿ⁺ each independently denotes an organic sulfonium cation, an organic sulfonium dication, an organic iodonium cation, an organic ammonium cation, an organic ammonium dication, an organic phosphonium cation, or an organic phosphonium dication,
C^{(am+bn)-} denotes a mono-lacunary Keggin-type or Dawson-type heteropolyacid anion, the heteropolyacid anion having a modified lacunary site, and
m denotes an integer in the range of 1 to 5, n denotes an integer of 1 or 2, and a is a real number]

The alkali metal ion, alkaline-earth metal ion, an organic sulfonium cation, an organic sulfonium dication, an organic iodonium cation, an organic ammonium cation, an organic ammonium dication, an organic phosphonium cation, and organic phosphonium dication may be the same as those described above.

C^{(am+bn)-} may denote a mono-lacunary Keggin-type or Dawson-type heteropolyacid anion having a modified lacunary site, which is represented by any one of the general formulae (VI-1) to (VI-8).

In the general formula (VII), m denotes an integer in the range of 1 to 5, n denotes an integer of 1 or 2, and a and b denote real numbers.

Furthermore, a mixture of heteropolyacid salts having a modified lacunary site may contain a plurality of heteropolyacid salts having a modified lacunary site differing in the ratio of A^{m+} to Bⁿ⁺. In this case, the values of a and b can be determined by, for example, NMR analysis, XRF analysis, XPS analysis, or the like.

The ratio of a to b depends on the types of the heteropolyacid anion having the modified lacunary site, A^{m+}, and Bⁿ⁺, and is preferably a:b = 0 to 3:0 to 12, more preferably 0 to 2:0 to 8, still more preferably 0 to 1:1 to 4.

In particular, when the value of a + b is a real number in the range of 3 to 7, and m is 1, it is preferable that a is a real number in the range of 0 to 3, and b is a real number in the range of 0 to 7, and it is more preferable that a is a real number in the range of 0 to 2, and b is a real number in the range of 2 to 7.

After the salt exchange reaction, the method for producing the heteropolyacid salt having a modified lacunary site represented by the general formula (VII) or a mixture thereof is, for example, but not limited to, a method for removing a by-product by extraction in a water tank, a method for precipitating a by-product as an inorganic salt followed by filtration, or a method for crystallizing the heteropolyacid salt having a modified lacunary site represented by the general formula (VII) or a mixture followed by filtration.

### [3-3. Step (3a')]

As another method for producing a heteropolyacid salt having a modified lacunary site or a mixture thereof according to the present embodiment, the step (3) may include the following step (3a') instead of the step (3a).

The step (3a') includes a step of substituting the cation moiety of the heteropolyacid salt having the modified lacunary site represented by the general formula (I) or a mixture thereof with a metal ion, an ammonium ion, or an organic ammonium cation by a salt exchange reaction, and a step of performing salt exchange with an onium salt.

The salt exchange reactions in the two steps make it possible to produce a heteropolyacid salt having a modified lacunary site or a mixture thereof with fewer impurities.

The metal ion is, for example, an alkali metal ion, such as Li⁺, Na⁺, K⁺, Rb⁺, or Cs⁺; an alkaline-earth metal ion, such as Be²⁺, Mg²⁺, Ca²⁺, Sr²⁺, or Ba²⁺; Al³⁺, or the like, preferably an alkali metal ion or an alkaline-earth metal ion, more preferably an alkali metal ion, still more preferably Na⁺, K⁺, Rb⁺, or Cs⁺. The organic ammonium cation may be the organic primary ammonium cation (NH₃(R^{5A})⁺)), organic secondary ammonium cation (NH₂(R^{5A})(R^{5B}))⁺), organic tertiary ammonium cation (NH(R^{5A})(R^{5B})(R^{5C})⁺), or organic quaternary ammonium cation (N(R^{5A})(R^{5B})(R^{5C})(R^{5D})⁺).

More specifically, a preferred method for producing a heteropolyacid salt having a modified lacunary site represented by the general formula (VII) or a mixture thereof includes a step of reacting a heteropolyacid salt having a modified lacunary site represented by the general formula (I) or a mixture thereof with a compound having a metal ion, an ammonium ion, or an organic ammonium cation represented by the general formula (XV-1) to perform salt exchange, and a step of salt-exchanging the resulting compound represented by the general formula (XV-2) with an onium salt.

(A^{p+})_{q}[C^{(p×q)-}] (I)

(A^{'+})(Y⁻)ₙ (XV-1)

(A^{'+})_{pxq}[C^{(pxq)-}] (XV-2)

[wherein
A^{p+} each independently denotes H⁺, a metal ion, or an ammonium ion,
A^{'+} each independently denotes a metal ion, an ammonium ion, or an organic ammonium cation,
C^{(pxq)-} denotes a mono-lacunary Keggin-type or Dawson-type heteropolyacid anion, the heteropolyacid anion having a modified lacunary site,
Y⁻ denotes a monovalent counter anion, and
p denotes an integer in the range of 1 to 5, and q denotes a real number]

(A^{m+})ₐ(Bⁿ⁺)_{b}(C^{(am+bn)-}) (VII)

[wherein
A^{m+} each independently denotes H⁺, a metal ion, or an ammonium ion,
Bⁿ⁺ each independently denotes an organic sulfonium cation, an organic sulfonium dication, an organic iodonium cation, an organic ammonium cation, an organic ammonium dication, an organic phosphonium cation, or an organic phosphonium dication,
C^{(am+bn)-} denotes a mono-lacunary Keggin-type or Dawson-type heteropolyacid anion, the heteropolyacid anion having a modified lacunary site, and
m denotes an integer in the range of 1 to 5, n denotes an integer of 1 or 2, a denotes a real number, and b denotes a real number greater than 0]

The compound represented by the general formula (XV-1) is, for example, sodium chloride, potassium chloride, rubidium chloride, cesium chloride, magnesium chloride, calcium chloride, strontium chloride, barium chloride, aluminum chloride, sodium bromide, potassium bromide, rubidium bromide, cesium bromide, magnesium bromide, calcium bromide, strontium bromide, barium bromide, aluminum bromide, sodium iodide, potassium iodide, rubidium iodide, cesium iodide, magnesium iodide, calcium iodide, strontium iodide, barium iodide, aluminum iodide, ammonium chloride, ammonium bromide, a salt of a halide ion with one exemplified above as the organic quaternary ammonium cation, or the like.

When the compound represented by the general formula (XV-1) is reacted with an onium salt, a salt exchange reaction can be performed using a two-phase system of an organic solvent and water. By using the two phase system of an organic solvent and water, the organic solvent layer contains the heteropolyacid salt having a modified lacunary site represented by the general formula (VII) or a mixture thereof, and a compound, such as a metal salt or an ammonium salt, corresponding to a by-product is mainly contained in the aqueous layer.

The organic solvent is, for example, methanol, ethanol, 1,4-dioxane, acetonitrile, 1-propanol, 2-propanol, ethyl acetate, acetonitrile, acetone, dichloromethane, chloroform, dimethylformamide, diethyl ether, or the like, preferably ethyl acetate, dichloromethane, chloroform, or diethyl ether.

### [3-4. Step (3b')]

As another method for producing a heteropolyacid salt having a modified lacunary site or a mixture thereof according to the present embodiment, the step (3) may include the following step (3b') subsequent to the step (3a) or the step (3a').

In the salt exchange reaction in the step (3a) or (3a'), the resulting by-product is removed by extraction into the aqueous layer, while the heteropolyacid salt having a modified lacunary site represented by the general formula (VII) or a mixture thereof is contained in the organic solvent layer. Furthermore, after the salt exchange reaction in the step (3a) or (3a'), the heteropolyacid salt having a modified lacunary site represented by the general formula (VII) or a mixture thereof can be precipitated and obtained by a recrystallization method or the like.

The step (3b') is a step of washing the heteropolyacid salt having a modified lacunary site represented by the general formula (VII) or a mixture thereof contained in the organic solvent layer or the precipitate with an acidic aqueous solution with a pH of 6 or less. The step (3b') makes it possible to obtain a heteropolyacid salt having a modified lacunary site or a mixture thereof with less impurities.

The pH of the acidic aqueous solution is preferably 6 or less, preferably 5.8 or less, more preferably -1 to 4.5, still more preferably -0.5 to 3.5, still more preferably 0 to 2.0.

The type of acid in the acidic aqueous solution is, for example, an inorganic acid, such as hydrochloric acid, sulfuric acid, nitric acid, sulfamic acid, or phosphoric acid; an organic acid having a carboxy group, such as tartaric acid, oxalic acid, formic acid, malic acid, citric acid, succinic acid, maleic acid, fumaric acid, glycolic acid, trifluoroacetic acid, or trichloroacetic acid; an organic acid having a sulfo group, such as methanesulfonic acid, ethanesulfonic acid, sulfosuccinic acid, m-toluenesulfonic acid, or p-toluenesulfonic acid; or the like. These acids may be used alone or in combination of two or more types thereof. The acid is preferably hydrochloric acid, sulfuric acid, tartaric acid, formic acid, citric acid, succinic acid, or malic acid.

The step of washing the heteropolyacid salt having a modified lacunary site represented by the general formula (VII) or a mixture thereof with an acidic aqueous solution with a pH of 6 or less may be followed by a step of washing the organic solvent layer or precipitate containing the heteropolyacid salt having a modified lacunary site or a mixture thereof multiple times with pure water or the like.

The step (3b') can remove metals or metal ions, such as Li, Na, Mg, Al, K, Ca, Ti, V, Cr, Mn, Fe, Co, Ni, Cu, Zn, As, Sr, Zr, Mo, Ag, Cd, Sn, Sb, Ba, W, Pb, and Au, contained as impurities in the heteropolyacid salt having a modified lacunary site represented by the general formula (VII) or a mixture thereof (excluding the metals or metal ions contained in the polyatom, the heteroatom, and A^{m+} of the polyacid salt or the mixture thereof), and can reduce any of the contents to less than 100 ppb.

The content of metals or metal ions contained as impurities in a heteropolyacid having a modified lacunary site or a mixture thereof can be measured by organic coupled plasma mass spectrometry (ICP-MS: Inductively Coupled Plasma Mass Spectrometry).

The mechanism by which the step of washing the heteropolyacid salt having a modified lacunary site represented by the general formula (VII) or a mixture thereof with an acidic aqueous solution with a pH of 6 or less can effectively reduce the content of metals or metal ions contained as impurities is not clear.

In general, it is known that a heteropolyacid anion is susceptible to various chemical changes in pH, ionic strength, redox potential, or the like, and these chemical changes affect the cluster structure/frame, formation mechanism, interaction network, and the like of the heteropolyacid anion.

For such a reason, as the mechanism, it is considered that exposure of the heteropolyacid salt having a modified lacunary site or a mixture thereof to an acidic aqueous solution with a pH of 6 or less can loosen the structure/frame, formation mechanism, interaction network, and the like of the heteropolyacid anion, making it possible to effectively wash out and remove metals or metal ions contained as impurities entangled in the structure/frame or the interaction network of the heteropolyacid anion.

### EXAMPLES

The present invention will be more specifically described below with reference to examples, but the present invention is not limited to these examples.

### [1. Production of Heteropolyacid Salt Having Modified Lacunary Site]

The heteropolyacid salts M-1-1, M-1-2, M-2, and M-3 having a modified lacunary site were produced by the following production method.

### [Production Example 1 (Example of Step (1)): Synthesis of Mono-Lacunary Potassium Silicotungstate (K₈[SiW₁₁O₃₉])]

380 g of 1 M acetic acid was added to 63 g of silicotungstic acid hydrate (H₄[SiW₁₂O₄₀]·xH₂O), and the reaction liquid was heated to 45°C. The pH was adjusted to 6.0 by addition of potassium hydrogen carbonate (52 g). The reaction liquid was then cooled to room temperature and was suction-filtered to prepare 56 g of a crude product. 216 g of pure water was added to the crude product, followed by heating to a water temperature of 73°C to redissolve the crude product. The solution was cooled at 4°C for 16 hours to recrystallize, and the resulting crystals were suction-filtered and dried under vacuum to obtain 34 g of the target compound.
²⁹Si-NMR (119.22 MHz, D₂O): δ (ppm) =-84.6 (s, 1Si).
¹⁸³W-NMR (20.84 MHz, D₂O): δ (ppm) =-101.85 (s, 2W), -116.26 (s, 2W), -119.29 (s, 1W), -125.62 (s, 2W), -140.85 (s, 2W), -174.53 (s, 2W).

### [Production Example 2-1 (Example of Steps (2) and (3)): Synthesis of Heteropolyacid Salt Having Modified Lacunary Site (Tetrakis(bis(2-trifluoromethylphenyl)phenylsulfonium)(1,3-divinyldisiloxan-1,1,3,3-tetrayl)undecatungstosilicate) (M-1-1)]

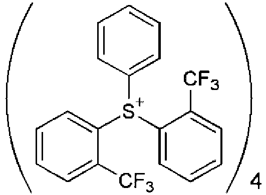

[SiW₁₁O₃₉(CH₂=CH-Si)₂O]⁴⁻

1.67 mL (10.9 mmol) of vinyltrimethoxysilane (CH₂=CH-Si(OCH₃)₃) was added to a mixed solvent of 402 g of acetonitrile and 184 g of pure water, and 10.9 g (3.6 mmol) of mono-lacunary potassium silicotungstate (K₈[SiW₁₁O₃₉]) synthesized above was added thereto. The pH was adjusted to 1.8 using 1 M hydrochloric acid. After stirring for 2 hours, the unreacted powder was suction-filtered, and the filtrate was concentrated to approximately 200 g using a rotary evaporator.

127 g (18.2 mmol) of a 6.3% aqueous solution of bis(2-trifluoromethylphenyl)phenylsulfonium chloride was added to the concentrate to precipitate a powder. The precipitated powder was suction-filtered and washed three times each with 180 mL of pure water, 180 mL of methanol, and 180 mL of methyl-t-butyl ether. The powder was then dispersed and washed with 160 g of methanol for 3 hours, was suction-filtered again, and was washed with 160 g of methanol three times. The powder was sufficiently dried under vacuum to produce 12.6 g of the target compound M-1-1 as a white powder.
¹H-NMR (400 MHz, DMSO-d6): δ (ppm) =5.94 (dd, 2H), 6.08 (dd, 2H), 6.13 (dd, 2H), 7.65-8.35 (m, 52H).
²⁹Si-NMR (119.22 MHz, DMSO-d6): δ (ppm) =-66.56 (s, 2Si), -85.29 (s, 1Si).
¹⁸³W-NMR (20.84 MHz, DMSO-d6): δ (ppm) =-105.52 (s, 2W), -107.54 (s, 2W), -112.04 (s, 1W), -126.08 (s, 2W), -170.95 (s, 2W), -247.66 (s, 2W).
ESI-MS: POSITIVE m/z 399.1 ([C₂₀H₁₃F₆S]⁺)
NEGATIVE m/z 700.3 (median) ([C₄H₆O₄₀Si₃W₁₁]⁴⁻)

### [Production Example 2-2 (Example of Step (3b')): Synthesis of Heteropolyacid Salt Having Modified Lacunary Site (Tetrakis(bis(2-trifluoromethylphenyl)phenylsulfonium)(1,3-divinyldisiloxan-1,1,3,3-tetrayl)undecatungstosilicate) (M-1-2)]

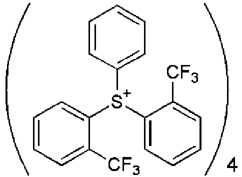

[SiW₁₁O₃₉(CH₂=CH-Si)₂O]⁴⁻

12.6 g of the M-1-1 powder obtained in Production Example 2-1 was dissolved in 50 mL of dichloromethane and was stirred and washed with 50 mL of 1% aqueous hydrochloric acid. The supernatant was then removed, and the residue was washed three times with 50 mL of pure water. 400 mL of methyl-t-butyl ether was then added to the resulting dichloromethane solution, and the precipitated powder was suction-filtered and washed with 400 mL of methyl-t-butyl ether. Furthermore, the powder was sufficiently dried under vacuum to obtain 11.3 g of the target compound M-1-2 as a white solid.

### [Production Example 3 (Example of Steps (2) and (3)): Synthesis of Heteropolyacid Salt Having Modified Lacunary Site ((Tetrakis(2-trifluoromethylbenzyl)triallylammonium)(1,3-divinyldisiloxan-1,1,3,3-tetrayl)undecatungstosilicate) (M-2)]

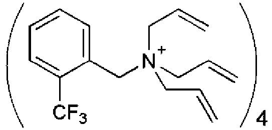

[SiW₁₁O₃₉(CH₂=CH-Si)₂O]⁴⁻

0.64 mL (1.4 mmol) of vinyltrimethoxysilane (CH₂=CH-Si(OCH₃)₃) was added to a mixed solvent of 165 g of acetonitrile and 71 g of pure water, and 4.2 g (4.2 mmol) of mono-lacunary potassium silicotungstate (K₈[SiW₁₁O₃₉]) synthesized above was added thereto. The pH was adjusted to 1.8 using 1 M hydrochloric acid. After stirring for 2 hours, the unreacted powder was suction-filtered, and the filtrate was concentrated to approximately 72 g using a rotary evaporator.

17 g (7.0 mmol) of a 20% aqueous solution of (2-trifluoromethylbenzyl)triallylammonium bromide was added to the concentrate to precipitate a powder. The precipitated powder was suction-filtered and washed three times each with 70 mL of pure water, 70 mL of methanol, and 70 mL of methyl-t-butyl ether. The powder was then dispersed and washed with 60 g of methanol for 3 hours, was suction-filtered again, and was washed with 60 g of methanol three times. The powder was sufficiently dried under vacuum to produce 4.5 g of the target compound M-2 as a white powder.
¹H-NMR (400 MHz, DMSO-d6): δ (ppm) =4.04 (d, 24H), 4.73 (s, 8H), 5.65 (d, 12H), 5.73 (d, 12H), 5.94 (d, 2H), 6.02-6.13 (m, 16H), 7.80-7.88 (m, 12H), 7.94 (d, 4H).
²⁹Si-NMR (119.22 MHz, DMSO-d6): δ (ppm) =-66.56 (s, 2Si), -85.29 (s, 1Si).
¹⁸³W-NMR (20.84 MHz, DMSO-d6): δ (ppm) =-105.52 (s, 2W), -107.54 (s, 2W), -112.04 (s, 1W), -126.08 (s, 2W), -170.95 (s, 2W), -247.66 (s, 2W).
ESI-MS: POSITIVE m/z 296.2 ([C₁₇H₂₁F₃N]⁺)
NEGATIVE m/z 700.3 (median) ([C₄H₆O₄₀Si₃W₁₁]⁴⁻)

### [Comparative Production Example 1 (Example Corresponding to Step (1)): Synthesis of Mono-Lacunary Potassium Silicotungstate (K₈[SiW₁₁O₃₉])]

Mono-lacunary potassium silicotungstate (K₈[SiW₁₁O₃₉]) was synthesized by the method described in Inorganic Syntheses, 1990, 27, 85-96 as described below.

94 g of pure water was added to 57.5 g of sodium tungstate dihydrate (Na₂WO₄·2H₂O) and was stirred at 80°C. 52 g of 4 M aqueous HCl was added dropwise to the reaction liquid, 4.5 g of an aqueous solution of sodium metasilicate nonahydrate (Na₂SiO₃·9H₂O) dissolved in 31 g of pure water was quickly added thereto, and 16 g of 4 M aqueous HCl was then added thereto. The reaction liquid was stirred at 60°C for 1 h and was cooled to room temperature. 47 g of KCl was then added thereto, and the resulting precipitate was suction-filtered. The precipitate was washed with 33 g of 1 M aqueous KCl and 33 g of cold water and was dried under vacuum to produce 26 g of the target compound.
²⁹Si-NMR (119.22 MHz, D₂O): δ (ppm) =-84.6 (s, 1Si).
¹⁸³W-NMR (20.84 MHz, D₂O): δ (ppm) =-97.43 (m, impurity), -101.85 (s, 2W), -116.26 (s, 2W), -119.29 (s, 1W), -125.62 (s, 2W), -140.85 (s, 2W), -174.53 (s, 2W).

### [Comparative Production Example 2 (Example Corresponding to Steps (2) and (3)): Synthesis of Heteropolyacid Salt Having Modified Lacunary Site (Tetrakis(bis(2-trifluoromethylphenyl)phenylsulfonium)(1,3-divinyldisiloxan-1,1,3,3-tetrayl)undecatungstosilicate) (M-3)]

3.8 g (1.3 mmol) of mono-lacunary potassium silicotungstate (K₈[SiW₁₁O₃₉]) synthesized above was added to 95g of pure water. After stirring, 0.97 mL (6.3 mmol) of vinyltrimethoxysilane (CH₂=CH-Si(OCH₃)₃) was added thereto, and 10.5 g of 1 M hydrochloric acid was added thereto and stirred for 20 hours. The unreacted powder was suction-filtered, and 53 g (7.6 mmol) of an aqueous solution of 6.3% bis(2-trifluoromethylphenyl)phenylsulfonium chloride was added thereto to precipitate a powder. The precipitated powder was poured into a suction funnel together with a small amount of pure water, was suction-filtered, and was washed with 151 g of isopropanol. The powder was sufficiently dried under vacuum to produce 5.0 g of the target compound M-3 as a white powder. It can be seen from the following NMR peaks of silicon and tungsten that impurities are contained.
¹H-NMR (400 MHz, DMSO-d6): δ (ppm) =5.94 (dd, 2H), 6.08 (dd, 2H), 6.13 (dd, 2H), 7.65-8.35 (m, 52H).
²⁹Si-NMR (119.22 MHz, DMSO-d6): δ (ppm) =-66.56 (s, 2Si), -69.74 (m, impurity), -85.29 (s, 1Si), -85.54 (s, impurity).
¹⁸³W-NMR (20.84 MHz, DMSO-d6): δ (ppm) =-91.48 (s, impurity), -105.52 (s, 2W), - 107.54 (s, 2W), -112.04 (s, 1W), -126.08 (s, 2W), -170.95 (s, 2W), -247.66 (s, 2W).
ESI-MS: POSITIVE m/z 399.1 ([C₂₀H₁₃F₆S]⁺)
NEGATIVE m/z 700.3 (median) ([C₄H₆O₄₀Si₃W₁₁]⁴⁻)

### <Synthesis Example 1: 3-(2-((1-methylcyclopentyl)oxycarbonyl)ethylthio)propyltrimethoxysilane>

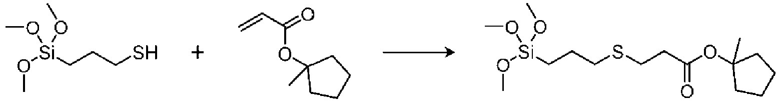

20 mL of methyl ethyl ketone was added to 7.7 g of 1-methylcyclopentyl acrylate and 9.8 g of 3-mercaptopropyltrimethoxysilane, and 0.3 g of 1,1'-azobis(cyclohexane-1-carbonitrile) was added thereto, followed by uniform stirring. Nitrogen was then blown thereinto for 30 minutes for nitrogen bubbling. The reaction liquid was heated from room temperature to 90°C over 30 minutes in nitrogen. The reaction liquid was kept at 90°C for 7 hours. Methyl ethyl ketone was distilled off from the reaction liquid using a rotary evaporator. The reaction liquid was sufficiently dried under vacuum to produce 16.6 g of the target compound as a colorless liquid.
¹H-NMR (400 MHz, DMSO-d6): δ (ppm) = 0.56 (t, 2H), 1.39 (s, 3H), 1.56-1.81 (m, 10H), 2.42 (t, 2H), 2.58 (t, 2H), 2.83 (t, 2H), 3.55 (s, 9H).

### [Production Example 4-1 (Example of Step (2) and First Half of Step (3a')): Synthesis of Heteropolyacid Salt Having Modified Lacunary Site (Tetrakis(benzyltriethylammonium)(1,3-di(3-(2-((1-methylcyclopentyl)oxycarbonyl)ethylthio)propyl)disiloxan-1,1,3,3-tetrayl)undecatungstosilicate) (M-4-1)]

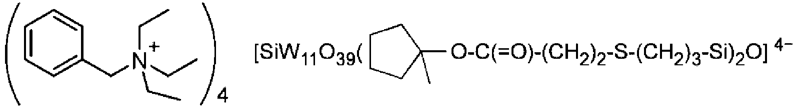

A target compound M-4-1 was produced in the same manner as in Production Example 2-1 except that, as raw material compounds, 3-(2-((1-methylcyclopentyl)oxycarbonyl)ethylthio)propyltrimethoxysilane was used instead of vinyltrimethoxysilane, and an aqueous solution of benzyltriethylammonium chloride was used instead of the aqueous solution of (2-trifluoromethylbenzyl)triallylammonium bromide.
¹H-NMR (400 MHz, DMSO-d6): δ (ppm) =0.67 (t, 4H), 1.34 (t, 36H), 1.49 (s, 6H), 1.54-1.71 (m, 16H), 1.99-2.08 (m, 4H), 2.49 (t, 4H), 2.58 (t, 4H), 2.67 (t, 4H), 3.20 (q, 24H), 4.49 (s, 8H), 7.52 (m, 20H).
²⁹Si-NMR (119.22 MHz, DMSO-d6): δ (ppm) =-53.02 (s, 2Si), -85.04 (s, 1Si).
ESI-MS: POSITIVE m/z 192.2 ([C₁₃H₂₂N]⁺)
NEGATIVE m/z 801.1 (median) ([C₂₄H₄₂O₄₄S₂Si₃W₁₁]⁴⁻)

### [Production Example 4-2 (Example of Second Half of Step (3a') and Step (3b')): Synthesis of Heteropolyacid Salt Having Modified Lacunary Site (Tetrakis(bis(2-trifluoromethylphenyl)phenylsulfonium)(1,3-di(3-(2-((1-methylcyclopentyl)oxycarbonyl)ethylthio)propyl)disiloxan-1,1,3,3-tetrayl)undecatungstosilicate) (M-4-2)]

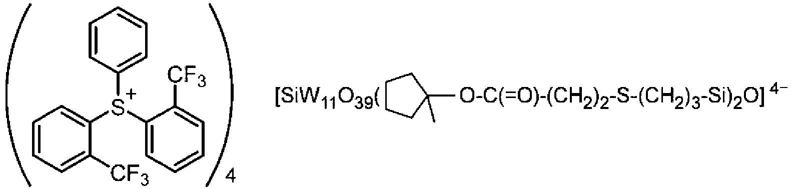

230 mL of dichloromethane was added to 14.5 g of the M-4-1 powder obtained in Production Example 4-1, and 264 g (18.2 mmol) of an aqueous solution of 3% bis(2-trifluoromethylphenyl)phenylsulfonium chloride was then added thereto. The product was then stirred at room temperature for 1 hour, and the aqueous layer was removed. Furthermore, in the same step, 264 g (2.9 mmol) of an aqueous solution of 0.5% bis(2-trifluoromethylphenyl)phenylsulfonium chloride was added thereto and was stirred for 10 minutes, and the aqueous layer was then removed. In the same step, washing was performed once with pure water, once with 1% hydrochloric acid, and three times with pure water, 400 mL of methyl-t-butyl ether was added to the resulting dichloromethane solution, and the precipitated powder was suction-filtered and was washed with 400 mL of methyl-t-butyl ether. Furthermore, the powder was sufficiently dried under vacuum to produce 15.7 g of the target compound M-4-2 as a white solid.
¹H-NMR (400 MHz, DMSO-d6): δ (ppm) =0.67 (t, 4H), 1.49 (s, 6H), 1.54-1.71 (m, 16H), 1.99-2.08 (m, 4H), 2.49 (t, 4H), 2.58 (t, 4H), 2.67 (t, 4H), 7.65-8.35 (m, 52H).
²⁹Si-NMR (119.22 MHz, DMSO-d6): δ (ppm) =-53.02 (s, 2Si), -85.04 (s, 1Si).
ESI-MS: POSITIVE m/z 399.1 ([C₂₀H₁₃F₆S]⁺)
NEGATIVE m/z 801.1 (median) ([C₂₄H₄₂O₄₄S₂Si₃W₁₁]⁴⁻)

### [Production Example 4-3 (Example of Second Half of Step (3a') and Step (3b')): Synthesis of Heteropolyacid Salt Having Modified Lacunary Site (Tetrakis(bis(2-trifluoromethylphenyl)phenylsulfonium)(1,3-di(3-(2-((1-methylcyclopentyl)oxycarbonyl)ethylthio)propyl)disiloxan-1,1,3,3-tetrayl)undecatungstosilicate) (M-4-3)]

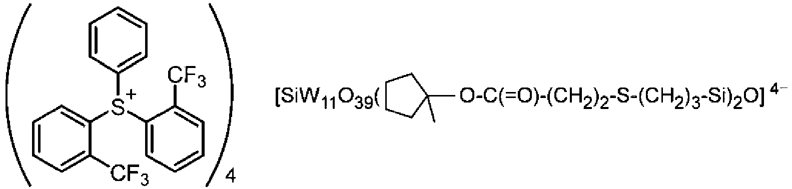

230 mL of dichloromethane was added to 14.5 g of the M-4-1 powder obtained in Production Example 4-1, and 264 g (18.2 mmol) of an aqueous solution of 3% bis(2-trifluoromethylphenyl)phenylsulfonium chloride was then added thereto. The product was then stirred at room temperature for 1 hour, and the aqueous layer was removed. Furthermore, in the same step, 264 g (2.9 mmol) of an aqueous solution of 0.5% bis(2-trifluoromethylphenyl)phenylsulfonium chloride was added thereto and was stirred for 10 minutes, and the aqueous layer was then removed. In the same step, washing was performed once with pure water, once with 5% citric acid, and three times with pure water, 400 mL of methyl-t-butyl ether was added to the resulting dichloromethane solution, and the precipitated powder was suction-filtered and was washed with 400 mL of methyl-t-butyl ether. Furthermore, the powder was sufficiently dried under vacuum to produce 15.4 g of the target compound M-4-3 as a white solid.

### [Production Example 5-1 (Example of Step (2) and First Half of Step (3a')): Synthesis of Heteropolyacid Salt Having Modified Lacunary Site (Tetrapotassium(1,3-di(4-methoxymethoxyphenyl)disiloxan-1,1,3,3-tetrayl)undecatungstosilicate) (M-5-1)]

(K⁺)₄ [SiW₁₁O₃₉(p-CH₃-O-CH₂-O-(C₆H₄)-Si)₂O]⁴⁻

The target compound M-5-1 was produced in the same manner as in Production Example 3 except that, as raw material compounds, 4-methoxymethoxyphenyltrimethoxysilane was used instead of methyltrimethoxysilane, and an aqueous solution of potassium chloride was used instead of the aqueous solution of (2-trifluoromethylbenzyl)triallylammonium bromide.
¹H-NMR (400 MHz, DMSO-d6): δ (ppm) = 3.37 (s, 6H), 5.22 (s, 4H), 7.05 (d, 4H), 7.66 (d, 4H).
²⁹Si-NMR (119.22 MHz, DMSO-d6): δ (ppm) = -64.72 (s, 2Si), -85.02 (s, 1Si).
ESI-MS: POSITIVE m/z 39.0 ([K]⁺)
NEGATIVE m/z 755.1 (median) ([C₁₆H₁₈O₄₄Si₃W₁₁]⁴⁻)

### [Production Example 5-2 (Example of Second Half of Step (3a') and Step (3b')): Synthesis of Heteropolyacid Salt Having Modified Lacunary Site (Tetrakis(bis(2-trifluoromethylphenyl)phenylsulfonium)(1,3-di(4-methoxymethoxyphenyl)disiloxan-1,1,3,3-tetrayl)undecatungstosilicate) (M-5-2)]

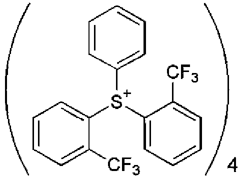

[SiW₁₁O₃₉(p-CH₃-O-CH₂-O-(C₆H₄)-Si)₂O]⁴⁻

230 mL of dichloromethane was added to 11.6 g of the M-5-1 powder obtained in Production Example 5-1, and 264 g (18.2 mmol) of an aqueous solution of 3% bis(2-trifluoromethylphenyl)phenylsulfonium chloride was then added thereto. The product was then stirred at room temperature for 1 hour, and the aqueous layer was removed. Furthermore, in the same step, 264 g (2.9 mmol) of an aqueous solution of 0.5% bis(2-trifluoromethylphenyl)phenylsulfonium chloride was added thereto and was stirred for 10 minutes, and the aqueous layer was then removed. In the same step, washing was performed once with pure water, once with 1% hydrochloric acid, and three times with pure water, 400 mL of methyl-t-butyl ether was added to the resulting dichloromethane solution, and the precipitated powder was suction-filtered and was washed with 400 mL of methyl-t-butyl ether. Furthermore, the powder was sufficiently dried under vacuum to produce 15.1 g of the target compound M-5-2 as a white solid.
¹H-NMR (400 MHz, DMSO-d6): δ (ppm) =3.37 (s, 6H), 5.22 (s, 4H), 7.05 (d, 4H), 7.65-8.35 (m, 56H).
²⁹Si-NMR (119.22 MHz, DMSO-d6): δ (ppm) =-64.72 (s, 2Si), -85.02 (s, 1Si).
ESI-MS: POSITIVE m/z 399.1 ([C₂₀H₁₃F₆S]⁺)
NEGATIVE m/z 755.1 (median) ([C₁₆H₁₈O₄₄Si₃W₁₁]⁴⁻)

### [2. Evaluation of Purity by NMR and Evaluation of Purity by Metal Content]

In ¹⁸³W-NMR and ²⁹Si-NMR of the heteropolyacid salts M-1-1, M-1-2, M-2, M-3, M-4-2, M-4-3, and M-5-2 having a modified lacunary site, observation of no impurities was evaluated as A, and observation of impurities was evaluated as B. Table 1 shows the results.

Furthermore, the amounts of metals or metal ions (Li, Na, Mg, Al, K, Ca, Ti, V, Cr, Mn, Fe, Co, Ni, Cu, Zn, As, Sr, Zr, Mo, Ag, Cd, Sn, Sb, Ba, W, Pb, and Au) in the heteropolyacid salts M-1-1, M-1-2, M-2, M-3, M-4-2, M-4-3, and M-5-2 having a modified lacunary site were measured with an inductively coupled plasma mass spectrometer (ICP-MS (Inductively Coupled Plasm Mass Spectrometry), Agilent 8900: manufactured by Agilent Technologies, Inc.).

For the metals or metal ions, when the content of the most abundant metal or metal ion other than tungsten was less than 100 ppb, it was evaluated as A, and when the content was 100 ppb or more, it was evaluated as B. Table 1 shows the results.

**[Table 1]**

| Heteropolyacid salt having modified lacunary site | Evaluation of purity by NMR | Evaluation of purity by metal content |
|---|---|---|
| M-1-1 | A | B |
| M-1-2 | A | A |
| M-2 | A | B |
| M-3 | B | B |
| M-4-2 | A | A |
| M-4-3 | A | A |
| M-5-2 | A | A |

From the above, when Production Example 1 and Comparative Production Example 1 are compared with each other, in Comparative Production Example 1, impurities were observed from the preparation stage of mono-lacunary potassium silicotungstate. Furthermore, also in the heteropolyacid salt M-3 having a modified lacunary site produced in Comparative Production Example 2, it is understood from the NMR peaks of silicon and tungsten that impurities different from the target compound are contained. These impurities were difficult to remove even by recrystallization or another technique.

On the other hand, in any of the mono-lacunary potassium silicotungstate and the heteropolyacid salts M-1-1, M-1-2, and M-2 having a modified lacunary site produced in Production Examples 1 to 3, peaks of impurities were not detected in NMR of silicon and tungsten.

Furthermore, when the heteropolyacid salt M-1-1 having a modified lacunary site obtained in Production Example 2-1 was compared with the heteropolyacid salt M-1-2 having a modified lacunary site obtained in Production Example 2-2, the heteropolyacid salt M-1-2 having a modified lacunary site had a lower content of metals or metal ions contained as impurities in the heteropolyacid salt having a modified lacunary site. This shows that the step (3b') of washing the heteropolyacid salt having a modified lacunary site with an acidic aqueous solution makes it possible to produce a higher-purity heteropolyacid salt having a modified lacunary site with less metal impurities.

Furthermore, when the heteropolyacid salt M-2 having a modified lacunary site obtained in Production Example 3 was compared with the heteropolyacid salt M-1-1 having a modified lacunary site obtained in Production Example 2-1, it was found that, although in both cases the content of the most abundant metal or metal ion other than tungsten was 100 ppb or more, the heteropolyacid salt M-2 having a modified lacunary site obtained by salt exchange with an organic ammonium salt contained less metal impurities than the heteropolyacid salt M-1-1 having a modified lacunary site obtained by salt exchange with an organic sulfonium salt, thereby providing a high-purity heteropolyacid salt having a modified lacunary site.

Thus, in Production Examples 4-1 and 4-2, after the steps (1) and (2), as the step (3a'), salt exchange with an ammonium salt was followed by salt exchange with an organic sulfonium salt, which is an onium salt. Furthermore, as the step (3b'), the heteropolyacid salt M-4-2 having a modified lacunary site, in which metal impurities were further reduced, was produced through a step of washing the heteropolyacid salt having a modified lacunary site with an acidic aqueous solution using hydrochloric acid.

Similarly, in Production Example 4-3, after the step (3a'), as the step (3b'), the heteropolyacid salt M-4-3 having a modified lacunary site, in which metal impurities were reduced, was produced through a step of washing the heteropolyacid salt having a modified lacunary site with an acidic aqueous solution using citric acid.

In Production Examples 5-1 and 5-2, after the steps (1) and (2), as the step (3a'), salt exchange with a potassium salt was followed by salt exchange with an organic sulfonium salt, which is an onium salt. Furthermore, as the step (3b'), the heteropolyacid salt M-5-2 having a modified lacunary site, in which metal impurities were further reduced, was produced through a step of washing the heteropolyacid salt having a modified lacunary site with an acidic aqueous solution using hydrochloric acid.

## Claims

1. A method for producing a heteropolyacid salt having a modified lacunary site or a mixture thereof, the method comprising:
(1) a step of converting a Keggin-type or Dawson-type heteropolyacid salt into a mono-lacunary Keggin-type or Dawson-type heteropolyacid salt; and
(2) a step of reacting the mono-lacunary Keggin-type or Dawson-type heteropolyacid salt with a P, Si, Ge, or Sn compound having one or more hydro groups or organic groups and two or more leaving groups to produce a heteropolyacid salt having a modified lacunary site represented by the general formula (1) or a mixture thereof.
(A^{p+})_{q}[C^{(pxq)-}] (I)
[In the formula (I),
A^{p+} each independently denotes H⁺, a metal ion, or an ammonium ion,
C^{(pxq)-} denotes a mono-lacunary Keggin-type or Dawson-type heteropolyacid anion, the heteropolyacid anion having a modified lacunary site, and
p denotes an integer in the range of 1 to 5, and q denotes a real number]

2. The method for producing a heteropolyacid salt having a modified lacunary site or a mixture thereof according to claim 1, wherein
the step (1) includes
(1a) a step of adjusting a pH of a solution containing the Keggin-type or Dawson-type heteropolyacid salt to 5.0 or less.

3. The method for producing a heteropolyacid salt having a modified lacunary site or a mixture thereof according to claim 1, wherein
the step (1) includes
(1a) a step of adjusting a pH of a solution containing the Keggin-type or Dawson-type heteropolyacid salt to 5.0 or less, and
(1b) a step of adjusting a pH of the solution to a range of 4 to 8 using a weak base or a base.

4. The method for producing a heteropolyacid salt having a modified lacunary site or a mixture thereof according to claim 1, wherein
the step (2) includes
(2a) a step of reacting the mono-lacunary Keggin-type or Dawson-type heteropolyacid salt with a P, Si, Ge, or Sn compound having one or more hydro groups or organic groups and two or more leaving groups in a solution with a pH of 5.0 or less.

5. The method for producing a heteropolyacid salt having a modified lacunary site or a mixture thereof according to claim 1, wherein the Keggin-type or Dawson-type heteropolyacid salt includes Mo, W, V, Nb, or Ta as a polyatom and P, Si, B, S, or Ge as a heteroatom.

6. The method for producing a heteropolyacid salt having a modified lacunary site or a mixture thereof according to claim 1, wherein the mono-lacunary Keggin-type or Dawson-type heteropolyacid salt is represented by the general formula (III-1) or (III-2).
[XM₁₁O₃₉]^{c21-} (III-1)
[X₂M₁₇O₆₁]^{c22-} (III-2)
[wherein
X denotes a heteroatom of P, Si, B, S, or Ge,
M denotes a polyatom of Mo, W, V, Nb, or Ta, and
c21 and c22 are natural numbers]

7. The method for producing a heteropolyacid salt having a modified lacunary site or a mixture thereof according to claim 1, wherein the organic group has one or more halogen atoms, haloalkyl groups, hydroxy groups, thiol groups, nitro groups, cyano groups, carboxy groups, amino groups, sulfo groups, epoxy groups, glycidyl groups, or amide groups.

8. The method for producing a heteropolyacid salt having a modified lacunary site or a mixture thereof according to claim 1, wherein the organic group has one or more ethylenically unsaturated double bonds.

9. The method for producing a heteropolyacid salt having a modified lacunary site or a mixture thereof according to claim 1, wherein the organic group has one or more hydroxy groups or carboxy groups, each having a protective group.

10. The method for producing a heteropolyacid salt having a modified lacunary site or a mixture thereof according to claim 1, wherein C^{(pxq)-} is represented by any one of the general formulae (VI-1) to (VI-8).
[XM₁₁O₃₉(R^{1A}X')₂O]^{c31-} (VI-1)
[XM₁₁O₃₉(R^{1B1}R^{1B2}Si)₂]^{c31-} (VI-2)
[XM₁₁O₃₉(R^{1C}P=O)₂]^{c31-} (VI-3)
[XM₁₁O₃₉(R^{1D}X")]^{c31-} (VI-4)
[X₂M₁₇O₆₁(R^{1A}X')₂O]^{c32-} (VI-5)
[X₂M₁₇O₆₁(R^{1B1}R^{1B2}Si)₂]^{c32-} (VI-6)
[X₂M₁₇O₆₁(R^{1C}P=O)₂]^{c32-} (VI-7)
[X₂M₁₇O₆₁(R^{1D}X")]^{c32-} (VI-8)
[wherein
X denotes a heteroatom of P, Si, B, S, or Ge,
M denotes a polyatom of Mo, W, V, Nb, or Ta,
X' denotes a heteroatom of Si or Ge,
X" denotes a heteroatom of Si, Ge, or Sn,
R^{1A} to R^{1D} each independently denote a hydro group, an optionally substituted C₁₋₁₈ hydrocarbyl group, an optionally substituted 3- to 18-membered non-aromatic heterocyclic group, or an optionally substituted 5- to 18-membered aromatic heterocyclic group,
a divalent carbon atom at any position of R^{1A} to R^{1D} except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{1A} to R^{1D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group, and
c31 and c32 are natural numbers]

11. The method for producing a heteropolyacid salt having a modified lacunary site or a mixture thereof according to claim 1, further comprising (3) a step of salt-exchanging the heteropolyacid salt having the modified lacunary site represented by the general formula (I) or a mixture thereof.

12. The method for producing a heteropolyacid salt having a modified lacunary site or a mixture thereof according to claim 11, wherein the step (3) includes (3a) a step of salt-exchanging the heteropolyacid salt having the modified lacunary site represented by the general formula (I) or a mixture thereof with an alkali metal salt, an alkaline-earth metal salt, an aluminum salt, or an onium salt.

13. The method for producing a heteropolyacid salt having a modified lacunary site or a mixture thereof according to claim 12, wherein the onium salt is an organic sulfonium salt, an organic iodonium salt, an organic ammonium salt, or an organic phosphonium salt.

14. The method for producing a heteropolyacid salt having a modified lacunary site or a mixture thereof according to claim 11, wherein the step (3) includes (3b) a step of obtaining a heteropolyacid salt having a modified lacunary site represented by the general formula (VII) or a mixture thereof.
(A^{m+})ₐ(Bⁿ⁺)_{b}(C^{(am+bn)-}) (VII)
[wherein
when b is 0,
A^{m+} each independently denotes H⁺, a metal ion, or an ammonium ion, at least one of which is an alkali metal ion, an alkaline-earth metal ion, or Al³⁺, different from A^{p+},
when b is a real number greater than 0,
A^{m+} each independently denotes H⁺, a metal ion, or an ammonium ion,
Bⁿ⁺ each independently denotes an organic sulfonium cation, an organic sulfonium dication, an organic iodonium cation, an organic ammonium cation, an organic ammonium dication, an organic phosphonium cation, or an organic phosphonium dication,
C^{(am+bn)-} denotes a mono-lacunary Keggin-type or Dawson-type heteropolyacid anion, the heteropolyacid anion having a modified lacunary site, and
m denotes an integer in the range of 1 to 5, n denotes an integer of 1 or 2, and a is a real number]

15. The method for producing a heteropolyacid salt having a modified lacunary site or a mixture thereof according to claim 11, wherein the step (3) includes (3a') a step of substituting a cation moiety of the heteropolyacid salt having the modified lacunary site represented by the general formula (I) or a mixture thereof with a metal ion, an ammonium ion, or an organic ammonium cation by a salt exchange reaction and a step of performing salt exchange with an onium salt.

16. The method for producing a heteropolyacid salt having a modified lacunary site or a mixture thereof according to claim 11, wherein the step (3) includes (3b') a step of washing a heteropolyacid salt having a modified lacunary site represented by the general formula (VII) or a mixture thereof with an acidic aqueous solution with a pH of 6 or less.
(A^{m+})ₐ(Bⁿ⁺)_{b}(C^{(am+bn)-}) (VII)
[wherein
when b is a real number greater than 0,
A^{m+} each independently denotes H⁺, a metal ion, or an ammonium ion,
Bⁿ⁺ each independently denotes an organic sulfonium cation, an organic sulfonium dication, an organic iodonium cation, an organic ammonium cation, an organic ammonium dication, an organic phosphonium cation, or an organic phosphonium dication,
C^{(am+bn)-} denotes a mono-lacunary Keggin-type or Dawson-type heteropolyacid anion, the heteropolyacid anion having a modified lacunary site, and
m denotes an integer in the range of 1 to 5, n denotes an integer of 1 or 2, a denotes a real number, and b denotes a real number greater than 0]
